# EUROPEAN PATENT SPECIFICATION

(11) **EP 1 502 538 B1**
(45) Date of publication and mention of the grant of the patent: **21.05.2008**
(21) Application number: 03812345.1
(22) Date of filing: 28.11.2003
(51) Int. Cl.: A61B 1/12

(54) **DEVICE AND METHOD FOR WASHING AND DISINFECTING ENDOSCOPE**
VORRICHTUNG UND VERFAHREN ZUM WASCHEN UND DESINFIZIEREN EINES ENDOSKOPS
DISPOSITIF ET PROCEDE POUR LE LAVAGE ET LA DESINFECTION D'UN ENDOSCOPE

(30) Priority: 29.11.2002 WO PCT/JP02/12546
(43) Date of publication of application: 02.02.2005
(73) Proprietor: Olympus Corporation, Tokyo 151-0072 (JP)
(72) Inventor: HASEGAWA, Hitoshi, Yokohama-shi, Kanagawa 222-0002 (JP)
(74) Representative: von Hellfeld, Axel
(86) International application number: PCT/JP2003/015246
(87) International publication number: WO 2004/049925

(56) References cited:
- EP-A- 0 878 165
- JP-A- 9 187 417
- JP-A- 2001 299 697
- JP-A- 2002 065 607
- US-A- 5 408 991
- PATENT ABSTRACTS OF JAPAN vol. 2002, no. 02, 2 April 2002 (2002-04-02) & JP 2001 299697 A (OLYMPUS OPTICAL CO LTD), 30 October 2001 (2001-10-30)

## Description

### TECHNICAL FIELD

The present invention relates to an endoscope cleaning and disinfecting device and to an endoscope cleaning and disinfecting method that are capable of cleaning and disinfecting internal channels of a used endoscope.

### BACKGROUND ART

Generally, endoscopes are utilized widely for medical checkup and treatment in a coelom, and various channels for sending air, sending water, and sucking them are incorporated with the endoscopes. Internal channels should be cleaned and disinfected each time endoscopes are used.

There are various kinds of endoscopes, such as ones for stomach, for duodenum, for large intestine, and for bronchia. Endoscopes for stomach generally have no special channels, but have only short channels with standard thickness. Endoscopes for duodenum have a forceps elevator wire channel in which a channel is remarkably thin. Endoscopes for large intestine have a long inserting unit and thick channels. On the contrary, endoscopes for bronchia have thin channels. The constitutions of the endoscope channels greatly vary according to types of endoscopes.

In order to enable various types of endoscopes to be cleaned and disinfected by one endoscope cleaning and disinfecting device, the cleaning and disinfecting device is connected with the endoscope channels at the time of cleaning and disinfecting, so that a cleaning liquid, a disinfectant, air, and the like are sent from the device into the endoscope channels. In order to keep cleaning and disinfecting properties in the endoscope channels, it is necessary to send liquids and air sufficiently to the endoscope channels at the time of the cleaning and disinfecting.

For example, DE 3918432 C2 discloses a conventional endoscope cleaning and disinfecting device that includes a channel clogging state monitor unit that measures a flow rate, pressure, and the like of liquid flowing through the endoscope channels so as to check if the endoscope channels have clogging and the liquid and air are sent sufficiently thereto.

Japanese patent application publication no. 2001-299697 discloses an earlier endoscope washing and disinfecting device of the current applicant. This device provides a flow sensor for measuring the flow rate of fluid in each duct during washing and disinfecting and a system for controlling the flow rate based on the measured value.

European patent application publication no. 0 878 165 describes a device and method for cleaning and disinfecting endoscopes in which the flow rate of cleaning fluid in all endoscope channels is limited by a flow restriction incorporated in each feed line.

As the air and liquid are sent into the endoscope channels, it has been difficult to detect whether they are supplied into endoscope channels with a suitable flow rate and pressure.

The present invention has been achieved according to the above circumstances, and it is an object to provide an endoscope cleaning and disinfecting device with a simple constitution that is capable of detecting a circulation state of air and liquid supplied into various endoscope channels.

### DISCLOSURE OF THE INVENTION

In order to achieve the object, the present invention provides an endoscope cleaning and disinfecting device having the features recited as in claim 1. The invention also provides a method of cleaning and disinfecting an endoscope having the steps recited in claim 17. Preferred features of the invention are recited in the dependent claims.

According to one aspect of the present invention, an endoscope cleaning and disinfecting device that cleans and disinfects an endoscope including plural types of channels having different fluid passing characteristics using a fluid, comprises: a fluid adjustment supplying unit that adjusts pressure or flow rates of the fluid to predetermined set values for each of the endoscope channels according to the fluid passing characteristics of the endoscope channels, and supplies the fluid to the endoscope channels; a measuring unit that measures the pressure or the flow rate of the fluid flowing through the endoscope channels; and a detecting unit that performs a comparison calculation based on measured values obtained by the measurement and the set values so as to detect clogging states of the endoscope channels,
wherein the set values are set so that the measuring unit can measure the pressure or the flow rates of the fluid.

According to the present invention, the pressure or the flow rates of the fluid to be supplied to the endoscope channels is adjusted to set values that can be measured by the measuring unit. This adjustment is made according to the fluid passing characteristics of the endoscope channels. Accordingly, the measuring unit can measure the pressure or the flow rates of the fluid flowing through the endoscope channels securely. Accordingly, measurement accuracy of the pressure or the flow rate of the fluid can be enhanced further in comparison with the case when the fluid is supplied to the endoscope channels without adjustment. For this reason, the clogging states of the endoscope channels can be detected accurately. The endoscope channels are ones provided with the endoscope, and include a suction channel, an air and water sending channel, a forceps elevator wire channel, and the like. The fluid passing characteristics of the endoscope channels include characteristics due to a diameter of the endoscope channels, an internal constitution, channel resistance, and the like. The fluid to be supplied to the endoscope channels is a fluid to be measured by the measuring unit, and includes a cleaning liquid, a rinsing liquid, a disinfectant, drying air, and the like. The measuring unit includes a pressure sensor, a flow rate sensor, and the like, and the measuring units may be of contact or non-contact type. The fluid adjustment supplying unit may include a supplying unit that supplies a fluid and an adjusting unit that adjusts the pressure or the flow rate of the fluid, or may include a single fluid adjustment supplying unit that can variably adjust the pressure or the flow rate of the fluid to be supplied. The set values may be fixed values or may be variable values controlled by feedback control. The set values and the measured values may be different kinds of values such as the pressure and the flow rates. In this case, either kind of values is converted into corresponding kind of values so as to be used for the comparison calculation.

According to another aspect of the endoscope cleaning and disinfecting device of the invention, when resistance of one endoscope channel is higher than that of another endoscope channel, the measuring unit adjusts the pressure of the fluid to be supplied to the endoscope channel having higher resistance, so that the pressure is adjusted higher than that of another endoscope channel.

It is generally difficult to detect a clogging state in the endoscope channel with high resistance based on the measurement of the flow rate, or an expensive sensor is required for the measurement of the flow rate. On this point, according to the invention, a fluid with higher pressure than that in the other endoscope channels is supplied to the endoscope channel with high resistance. Accordingly, since the supply amount of the fluid to the endoscope channel can be increased, the flow rate of the fluid can be measured suitably by an inexpensive sensor. The resistance in the channel is higher than that of the other channels means that, at the detection of the clogging states, for example, presence of clogging in the endoscope channels can be detected by measuring the flow rates, but it is difficult to detect a degree of clogging. The endoscope channels include, for example, the forceps elevator wire channel.

In the endoscope cleaning and disinfecting device according to another aspect of the present invention, when resistance of one endoscope channel is higher than that of another endoscope channel, the measuring unit for the endoscope channel having higher pressure is a pressure measuring unit that measures the pressure of the fluid.

According to the present invention, the pressure of the fluid in the endoscope channel with high resistance is measured. Accordingly, when the flow rate of the fluid supply is small, a degree of clogging in the endoscope channel can be detected.

In the endoscope cleaning and disinfecting device according to another aspect of the present invention, the set values are set so as to be equal to or less than a durability limit of the endoscope channels.

According to the present invention, the pressure or the flow rates of the fluid to be supplied are set so as to be equal to or less than the durability limit in the endoscope channels. Accordingly, the breakage of the endoscope channels caused by excessive supply of the fluid can be effectively prevented.

In the endoscope cleaning and disinfecting device according to another aspect of the present invention, the fluid adjustment supplying unit includes: a supplying unit that supplies the fluid; and an adjusting unit that is arrange on a channel of the fluid and adjusts the pressure or the flow rate of the passing fluid individually according to the fluid passing characteristics of the endoscope channels.

According to the present invention, the supplying unit that supplies the fluid and the adjusting unit that adjusts the pressure or the flow rates of the fluid are provided separately. The adjusting unit adjusts the pressure of the fluid or the flow rates of the fluid individually according to the fluid passing characteristics of the various endoscope channels. Accordingly, a pump whose output is constant can be used so as to adjust the pressure or the flow rates of the fluid in a simple constitution. The adjusting unit is provided with, for example, a plurality of relief valves that are provided on the branched channels led to the endoscope channels and that are set so as to have specific set values according to the fluid passing characteristics of the endoscope channels. Since the adjusting unit does not require a control system that controls the set values, it can be constituted simply. The adjusting unit includes, for example, a single relief valve that is provided on an upper stream of the branched channels and that can adjust the pressure or the flow rates of the fluid allowed to pass by electric control. Since the adjusting unit is provided with only the relief valve, the installation space for the adjusting unit can be reduced.

In the endoscope cleaning and disinfecting device according to another aspect of the present invention, the fluid adjustment supplying unit is a variable supplying unit that is capable of variably controlling the pressure or the flow rate of the fluid to be supplied according to the fluid passing characteristics of the endoscope channels.

According to the present invention, the fluid adjustment supplying unit is constituted by a variable supplying unit that can variably control outputs according to the fluid passing characteristics in the endoscope. Accordingly, the relief value can be omitted.

Since the flow rate of the fluid is smaller in the endoscope channel, such as the forceps elevator wire channel, with higher resistance than that of the other channels, its clogging state may not be detected in the measurement of flow rate. According to the invention, therefore, the pressure of the fluid is measured in the endoscope channel with higher resistance to detects clogging state. Accordingly, the measurement accuracy can be enhanced regardless of the flow rate of the fluid.

In the endoscope cleaning and disinfecting device according to another aspect of the present invention, the detecting unit includes a notifying unit that determines whether the measured values are within a predetermined range of the set values and notifies determined results gradually.

According to the present invention, the detected clogging state of the endoscope channels is determined based on the measured pressure or the flow rates of the fluid on a step-by-step basis, and the state is notified gradually. Accordingly, not only the presence of clogging in the endoscope but also a degree of clogging can be notified to the user, so that the reliability of the detected results can be enhanced.

In the endoscope cleaning and disinfecting device according to another aspect of the present invention, the detecting unit has a reading unit that reads the set values and the detecting unit performs the comparison calculation using the read set values.

According to the present invention, the set values of the fluid to be supplied are read, and the comparison calculation is performed by using the set values.

Accordingly, a circulation state of the air or the liquid to be supplied to various endoscope channels can be detected in a simple constitution.

The endoscope cleaning and disinfecting device according to another aspect of the present invention, the set values are preset in the endoscope to be cleaned and disinfected, and the detecting unit has a reading unit that reads the set values and the detecting unit performs the comparison calculation using the read set values.

According to the present invention, the set values of the fluid to be supplied are preset in the endoscope to be cleaned and disinfected, and the set values are read so that the comparison calculation are performed. Accordingly, even when the set values differ according to each endoscope, the set values specific to the endoscope are automatically determined, so that the clogging states can be detected.

The endoscope cleaning and disinfecting device according to another aspect of the present invention, the fluid adjustment supplying unit has an air sending source that sends air to the endoscope channels, a cleaning tube that is connected with the endoscope channels, a changeover unit that switches the endoscope channels to which the air is sent, and a unit that connects the air sending channel and the water sending channel in the endoscope.

The endoscope cleaning and disinfecting device according to another aspect of the present invention, the detecting unit includes a notifying unit that, when the measured values are within a predetermined range of the set values, notifies the state. According to the present invention, when the measured values are within a predetermined range of the set values according to the result of the comparison calculation, this is notified. Accordingly, the states of various endoscope channels can be checked accurately.

The endoscope cleaning and disinfecting device according to another aspect of the present invention, the contents to be notified includes states such as the flow rate decreases in a certain place of the endoscope channels, a cleaning tube is disconnected, the cleaning tube is forgotten to be attached, piping in the device is disconnected, a leakage occurs in the piping in the device, or the air sending source in the device is abnormally operated.

The endoscope cleaning and disinfecting device according to another aspect of the present invention, the fluid to be supplied to the endoscope channels is a liquid for cleaning and disinfecting, the fluid adjustment supplying unit has a water sending piping that leads the liquid to the endoscope channels, and a control unit that controls the liquid flowing through the endoscope channels based on the comparison calculation results is further provided.

The endoscope cleaning and disinfecting device according to another aspect of the present invention, a notifying unit notifies a control state of the control unit based on the compared result of the comparing unit.

The endoscope cleaning and disinfecting device according to another aspect of the present invention, a first connector connected detachably with an opening at one end of the first endoscope channel, a second connector that can be connected detachably with an opening at the other end of the first endoscope channel, a third connector that can be connected detachably with an opening at one end of the second endoscope channel, and a connecting unit that connects the second connector and the third connector, these units being provided on the water sending piping.

The endoscope cleaning and disinfecting device according to another aspect of the present invention comprises a first liquid sending piping connected with an end of the first endoscope channel and an end of the water sending piping, a second liquid sending piping connected with an end of the second endoscope channel and an end of the water sending piping, a plurality of electromagnetic valves provided on the first liquid sending piping and the second liquid sending piping respectively, and a control unit that selectively switches the electromagnetic valves.

The endoscope cleaning and disinfecting method according to one aspect of the present invention, which is an endoscope cleaning and disinfecting method of cleaning and disinfecting an endoscope including plural types of channels having different fluid passing characteristics using a fluid, comprises: a fluid adjustment supplying step of adjusting pressure or flow rates of the fluid to predetermined set values for each of the endoscope channels according to the fluid passing characteristics of the endoscope channels, and supplies the fluid to the endoscope channels; a measuring step of measuring the pressure or the flow rate of the fluid flowing through the endoscope channels; and a detecting step of performing a comparison calculation based on measured values obtained by the measurement and the set values so as to detect clogging states of the endoscope channels, wherein the set values are set so that the measuring unit can measure the pressure or the flow rates of the fluid.

According to the present invention, the pressure or the flow rates of the fluid to be supplied to the endoscope channels are adjusted to set values that can be measured by the measuring unit. This adjustment is made according to the fluid passing characteristics of the endoscope channels. The measuring unit can measure the pressure or the flow rates of the fluid flowing through the endoscope channels accurately. Accordingly, the measurement accuracy of the pressure or the flow rates of the fluid can be enhanced further in comparison with the case where the fluid is supplied to the endoscope channels without adjustment. For this reason, the clogging states of the endoscope channels can be detected accurately.

The endoscope cleaning and disinfecting method according to another aspect of the present invention, the detecting step is executed prior to cleaning and disinfecting steps for the endoscope channels.

According to the present invention, the clogging states of the endoscope channels are detected prior to the cleaning and disinfecting steps for the endoscope channels. Accordingly, the cleaning and disinfecting steps for a clogged endoscope channel can be omitted.

The endoscope cleaning and disinfecting method according to still another aspect of the present invention comprises a durability limit cleaning step of, when clogging of the endoscope channels is within a predetermined range as a result of detecting the clogging states, heightening the pressure or the flow rates of the fluid to be supplied to the endoscope channels up to a durability limit of the endoscope channels and of cleaning the endoscope channels.

The endoscope channel may be broken, if the pressure or the flow rate of the fluid for cleaning is raised to the durability limit of the endoscope channel when an endoscope channel is clogged. According to the invention, therefore, when the clogging of the endoscope channel is within a predetermined range according to a detection result of the clogging state, the pressure or the flow rate of the fluid is raised to the durability limit of the endoscope channel, and the endoscope channel is cleaned. Accordingly, the breakage of the endoscope channel is prevented, and simultaneously the endoscope channel can be cleaned within the durability limit. The durability limit may be changed according to clogging states of the endoscope channels.

The endoscope cleaning and disinfecting method according to another aspect of the present invention, the detecting step includes a reading step of reading the set values, and the comparison calculation is executed by using the read set values at the detecting step.

According to the present invention, the set values of the fluid to be supplied are read, and the comparison calculation are performed by using the set values. Accordingly, the circulation state of the air and the liquid to be supplied to various endoscope channels can be detected in a simple constitution.

The endoscope cleaning and disinfecting method according to another aspect of the present invention, the detecting step includes a notifying step of, when the measured values are within a predetermined range of the set values, notifying the state.

According to the present invention, when the measured values are within a range of the set values as a result of the comparison calculation, this is notified and the states of various endoscope channels can be checked accurately.

### BRIEF DESCRIPTION OF THE DRAWINGS

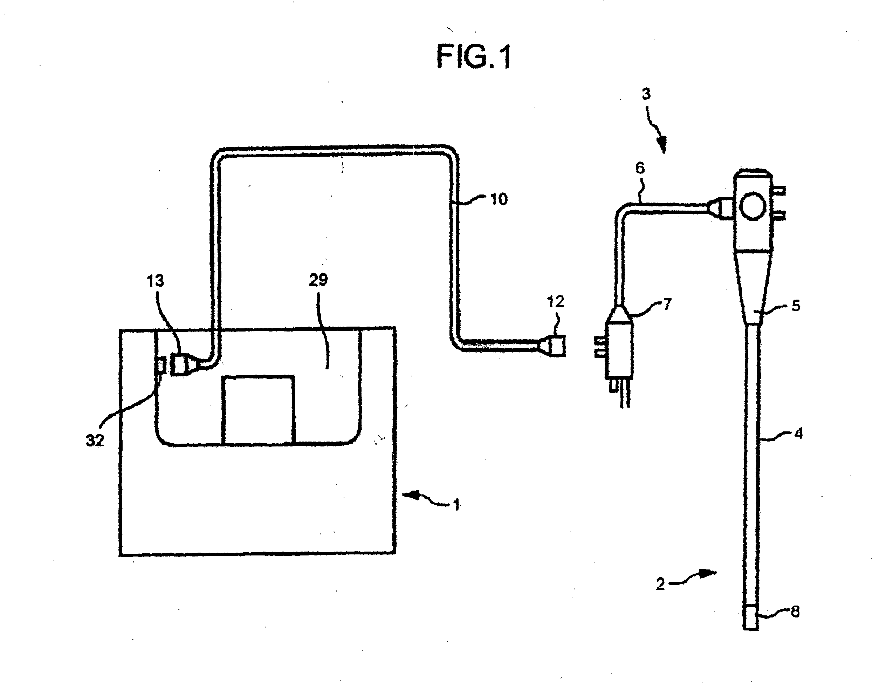

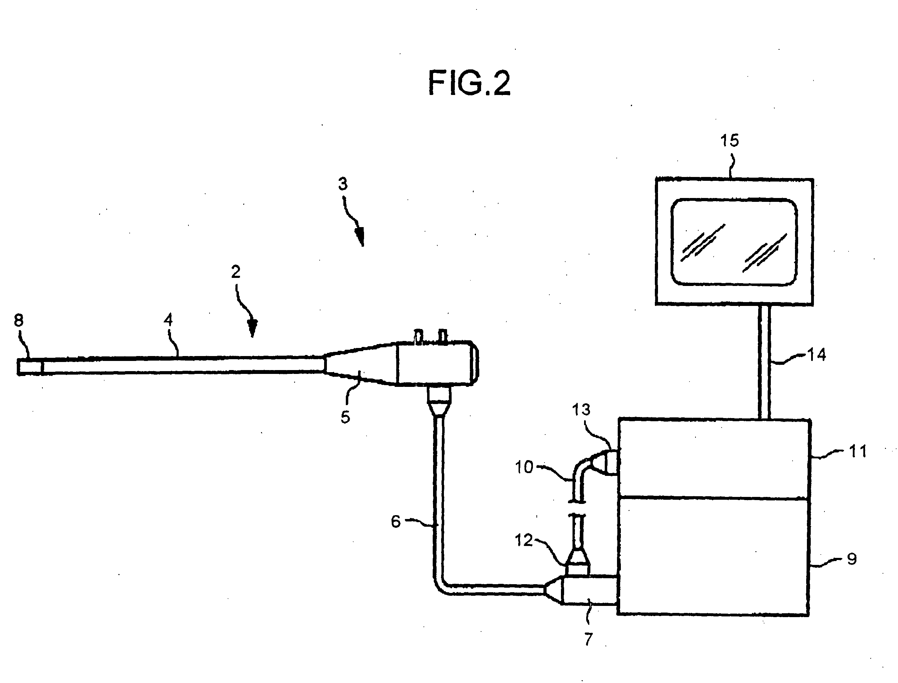

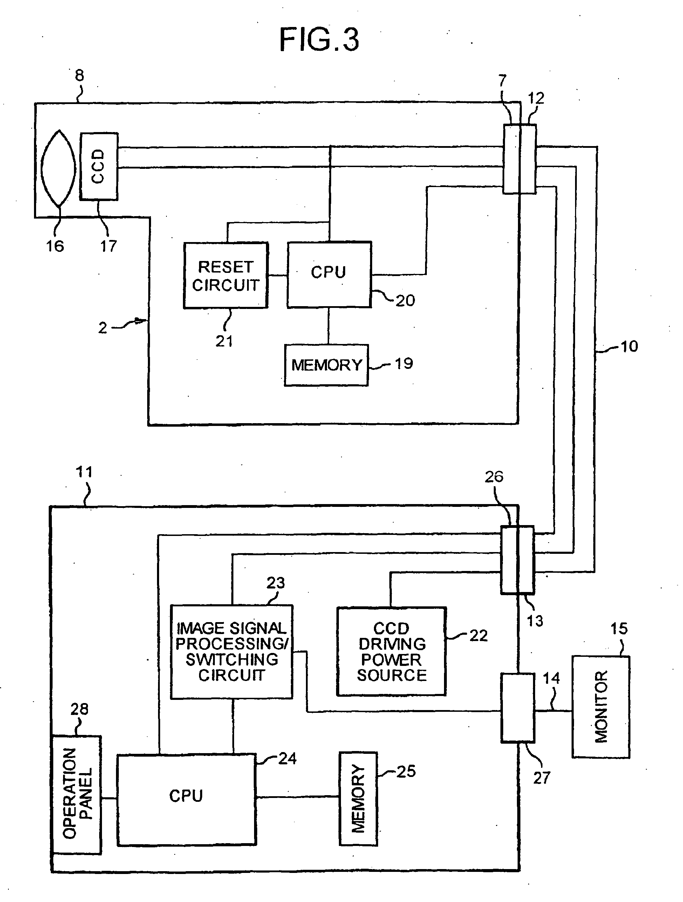

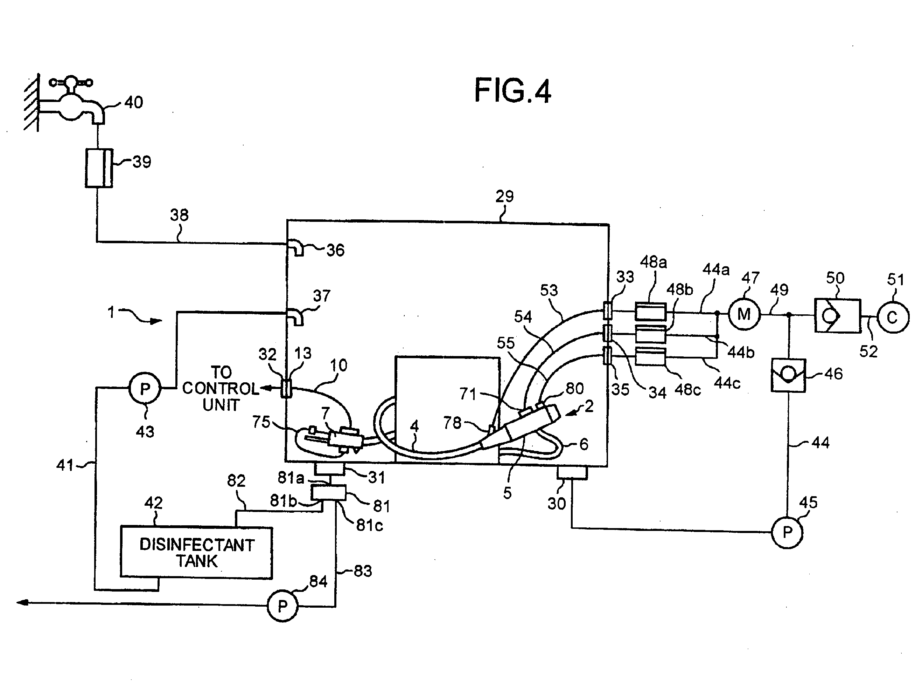

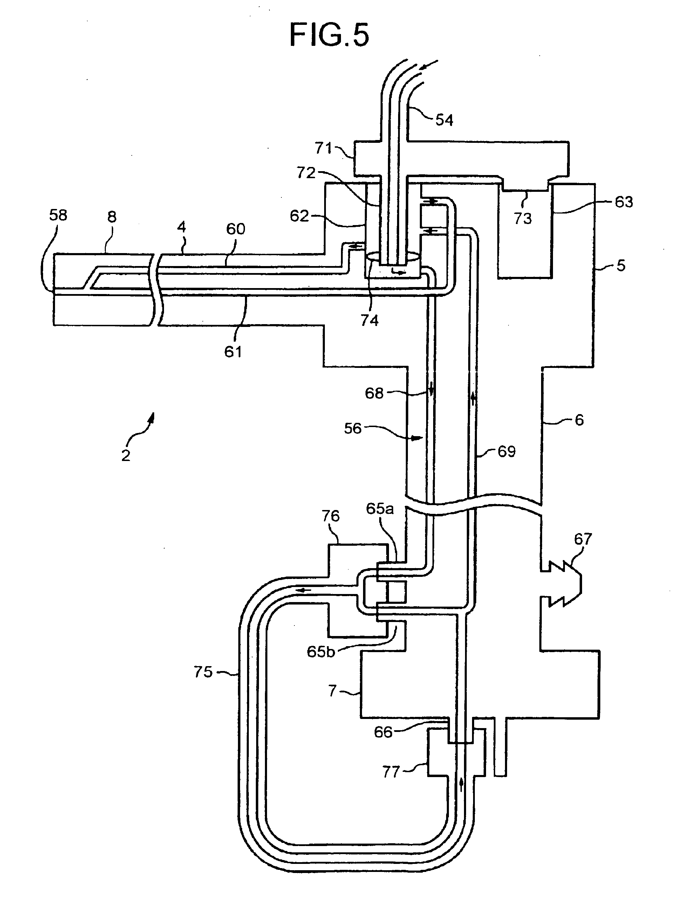

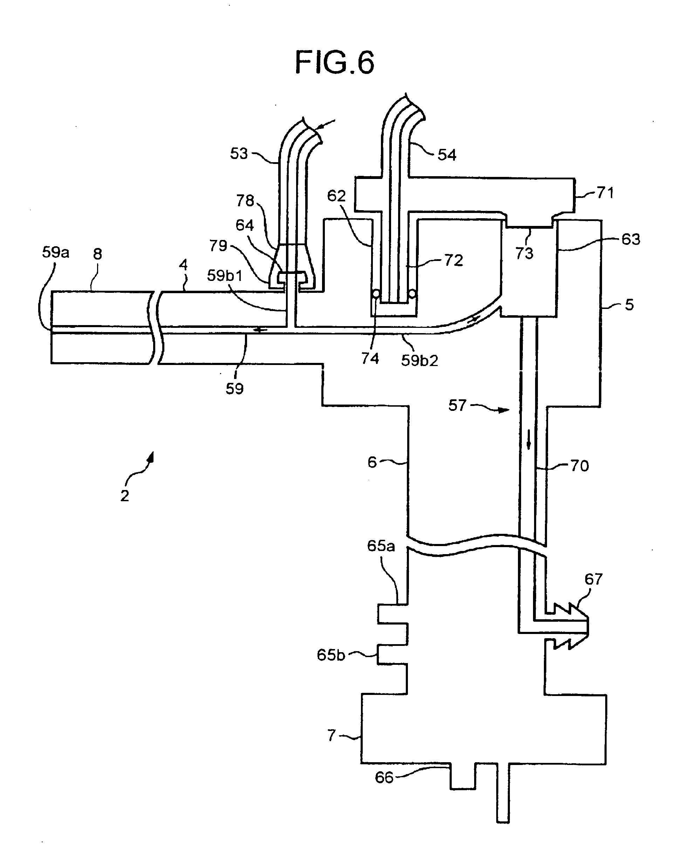

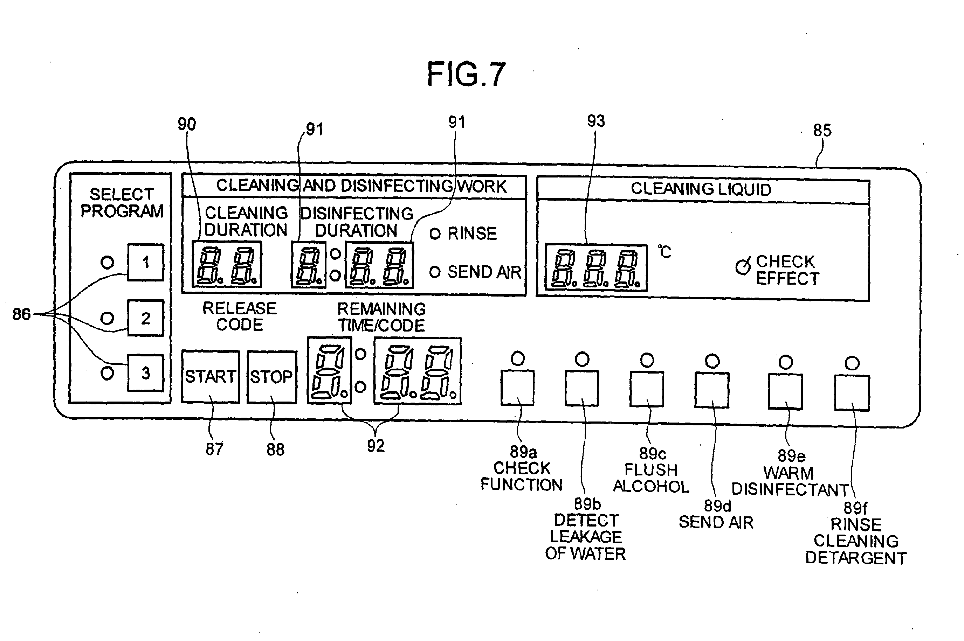

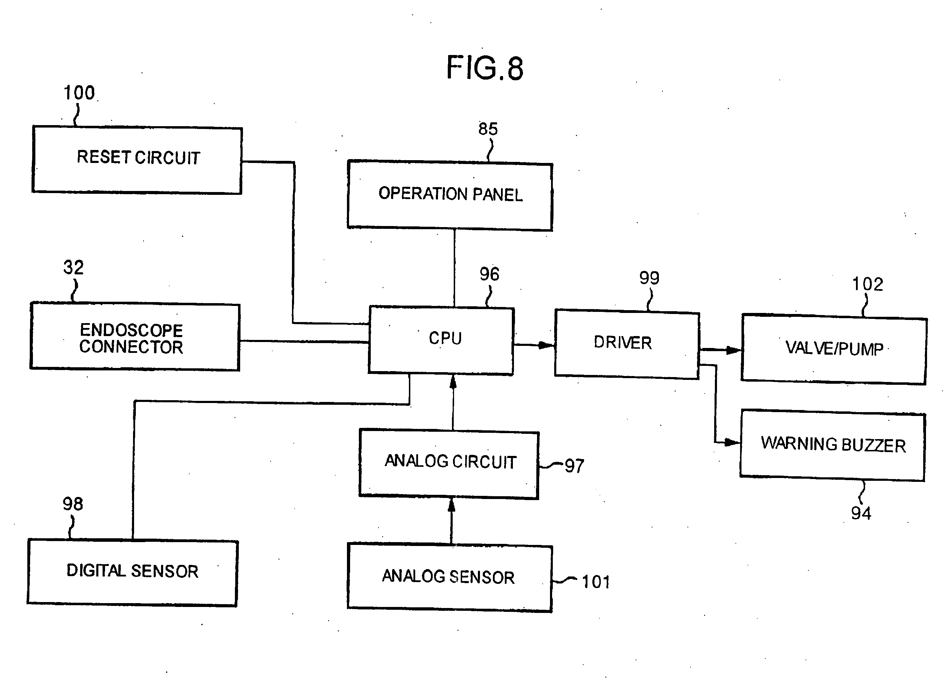

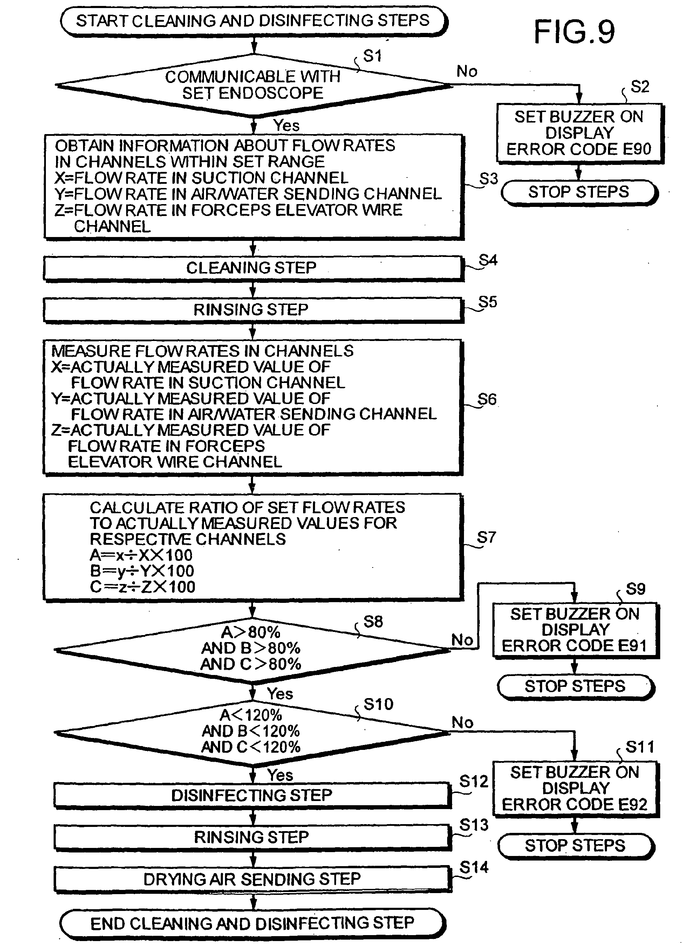

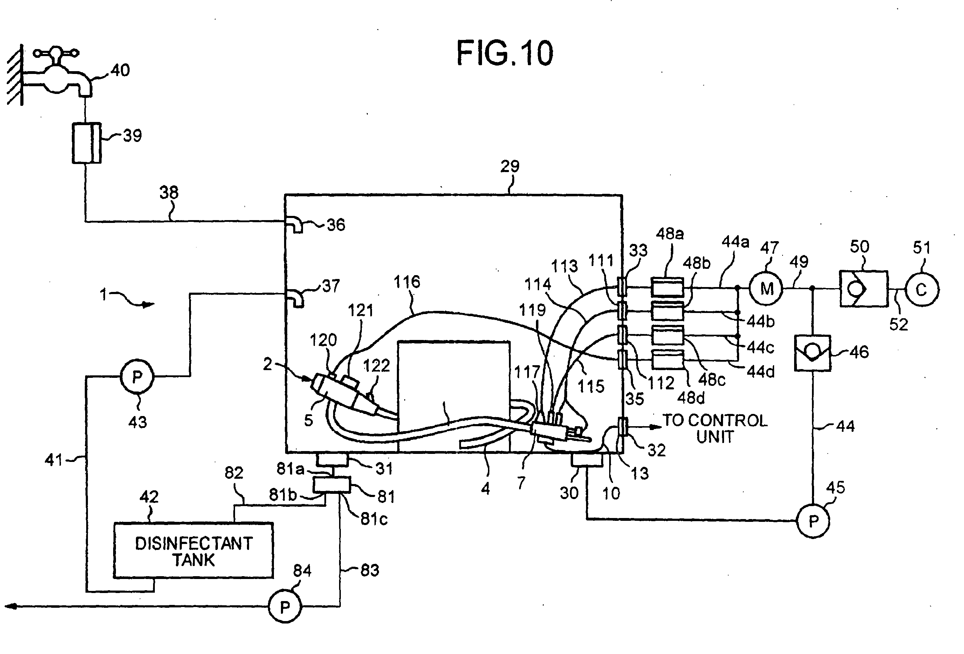

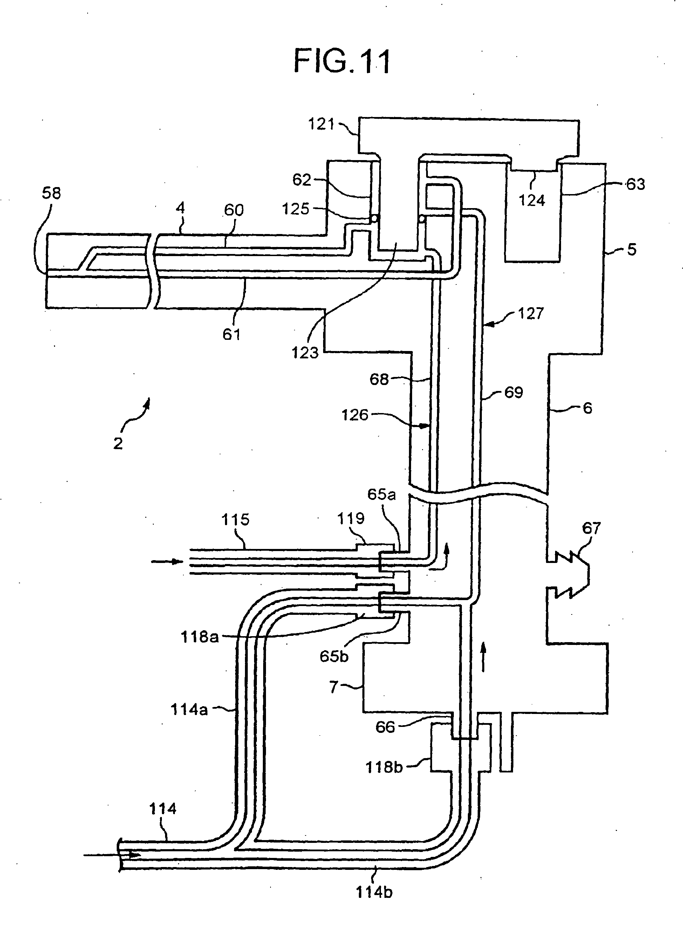

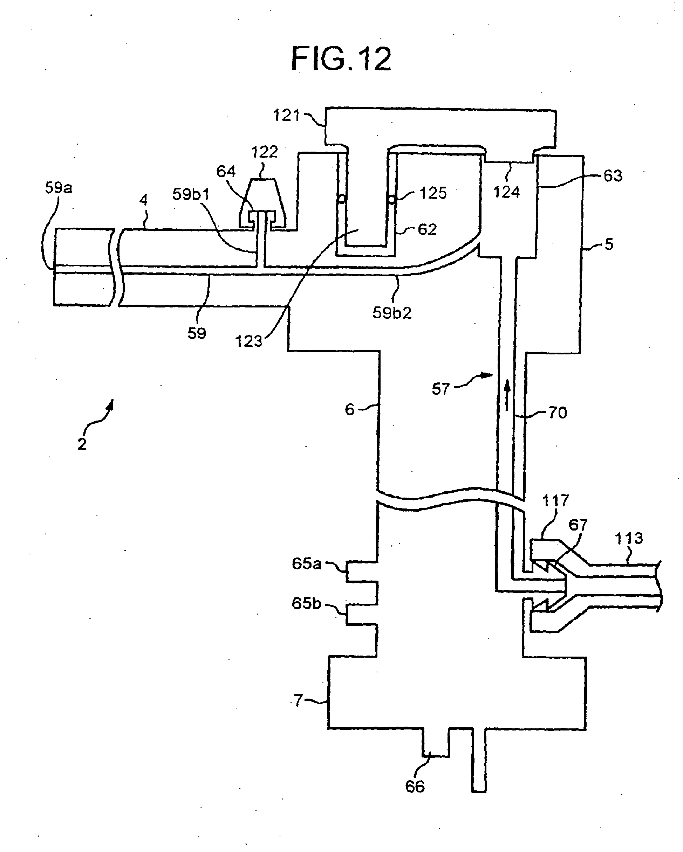

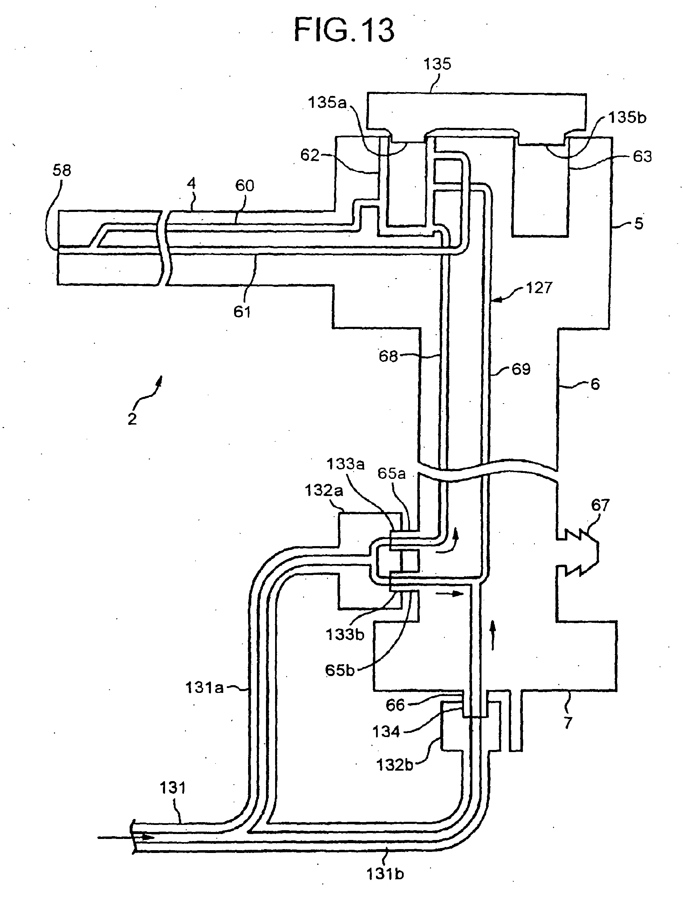

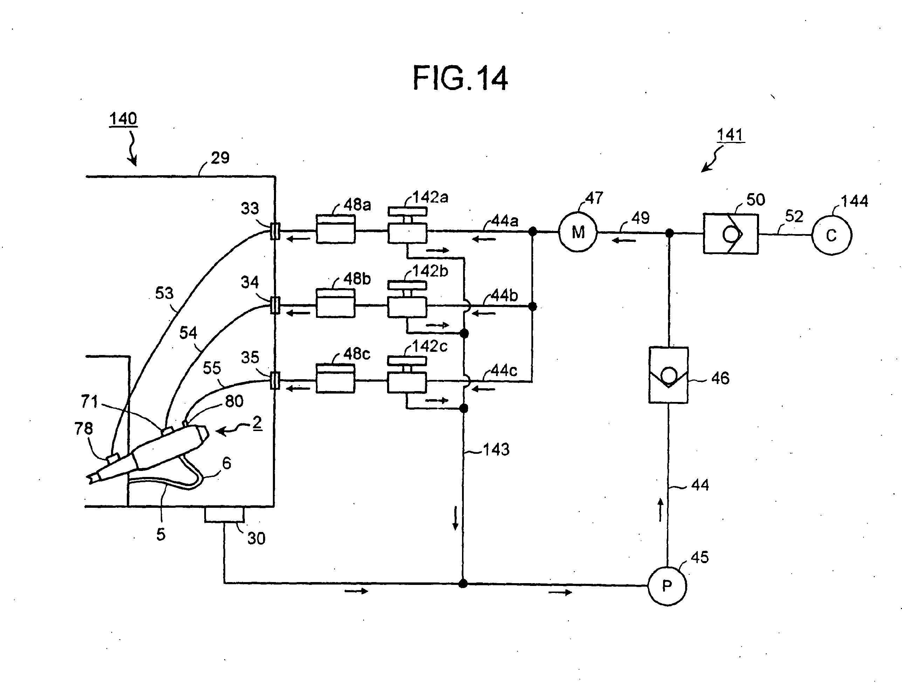

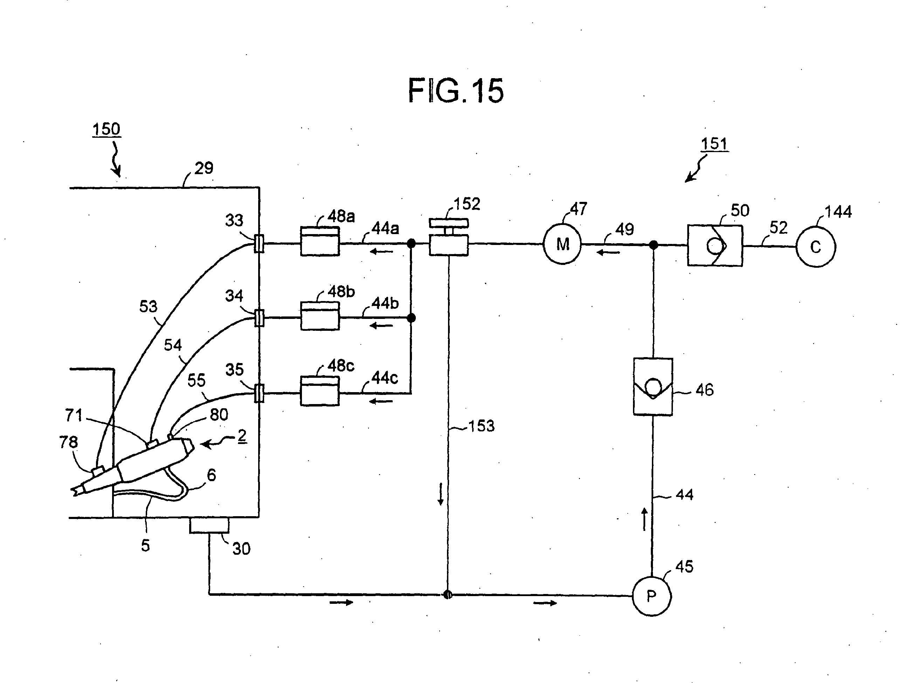

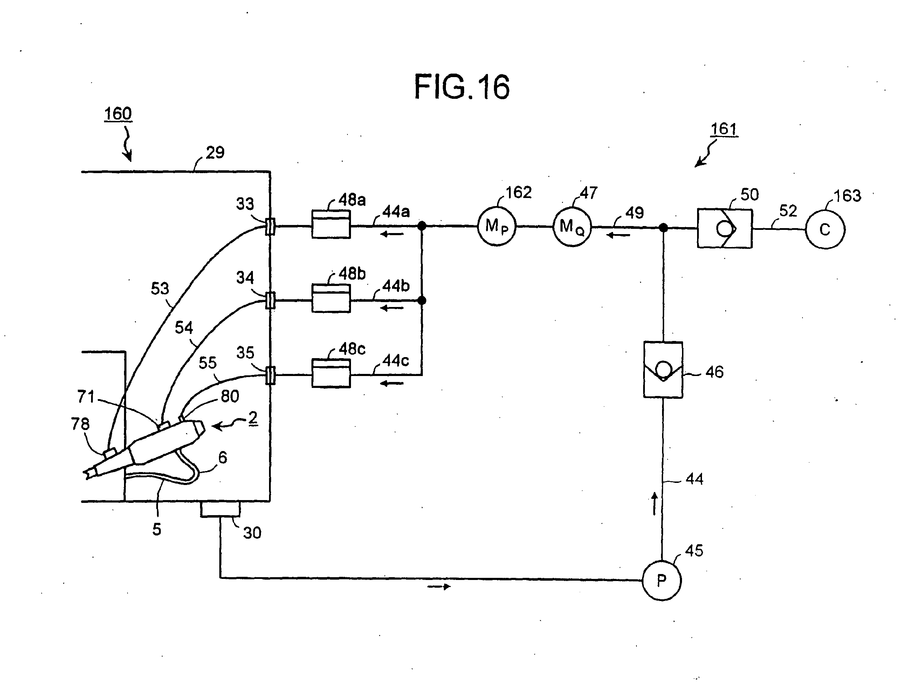

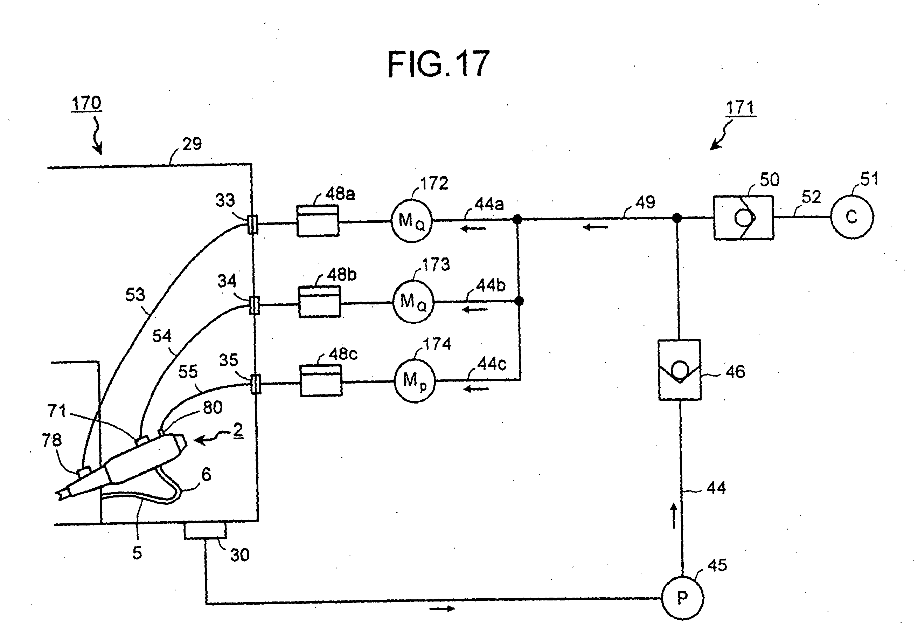

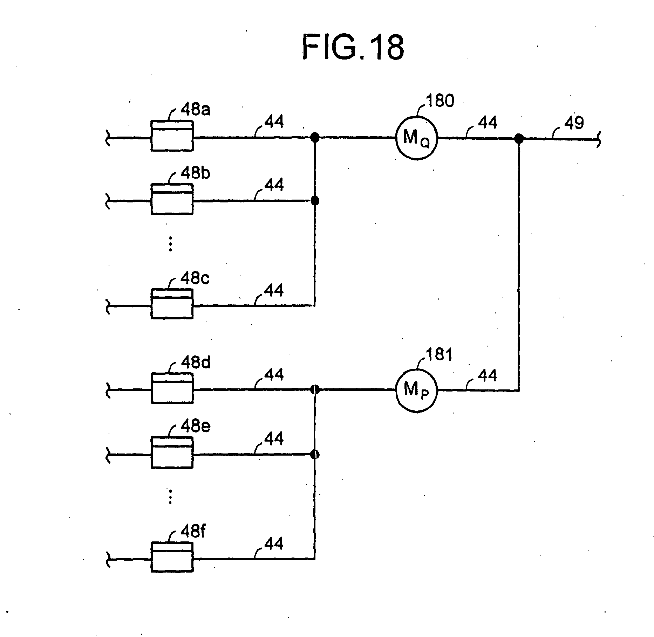

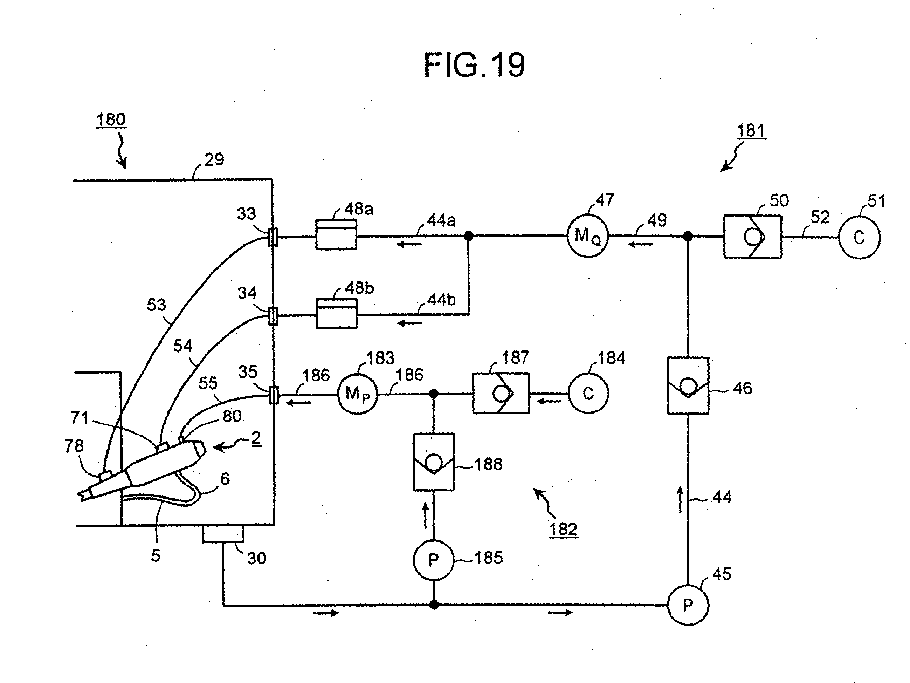

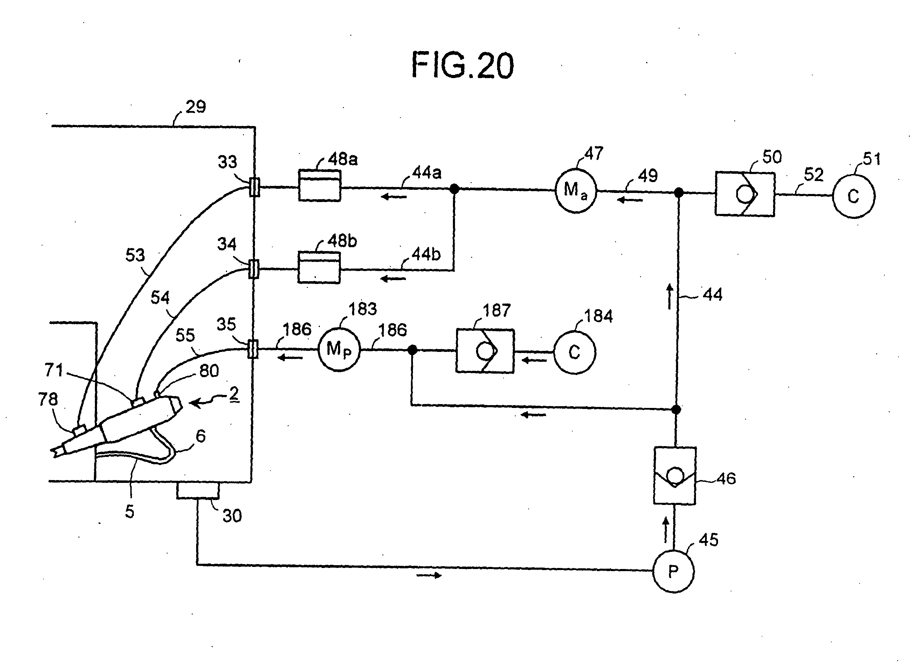

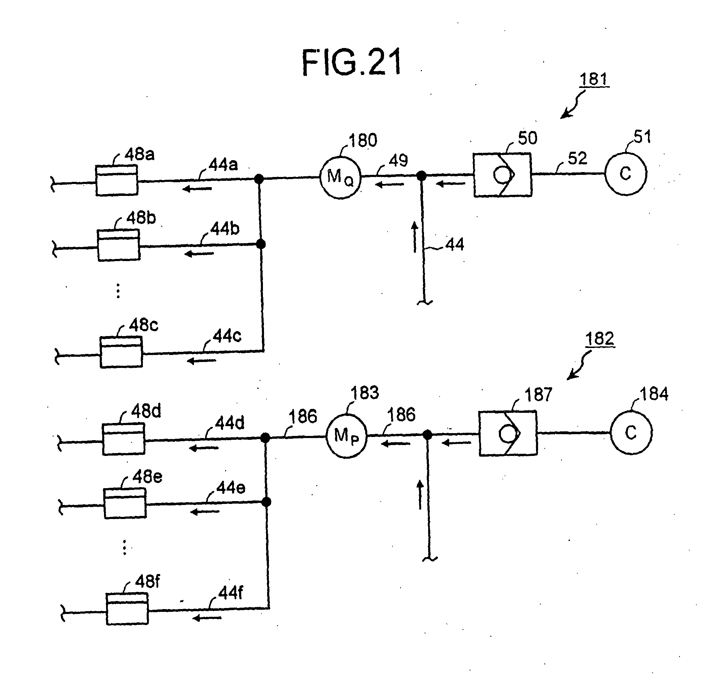

Fig. 1 is a schematic constitutional diagram of an endoscope cleaning and disinfecting device and an endoscope to be cleaned and disinfected according to a first embodiment of the present invention; Fig. 2 is a schematic constitutional diagram of an entire system of an endoscope device with which the endoscope to be cleaned and disinfected by the endoscope cleaning and disinfecting device according to the first embodiment is incorporated; Fig. 3 is a schematic constitutional diagram of the endoscope according to the first embodiment and an image processing device; Fig. 4 is a schematic constitutional diagram of an entire system of the endoscope cleaning and disinfecting device according to the first embodiment; Fig. 5 is a schematic constitutional diagram of a state that an air and water sending channel in the endoscope is connected with a bypass tube and a cleaning tube for cleaning and disinfecting a water sending channel when the endoscope cleaning and disinfecting device according to the first embodiment is used; Fig. 6 is a schematic constitutional diagram of a state that a suction channel in the endoscope is connected with a cleaning tube for cleaning and disinfecting the suction channel and the cleaning tube for cleaning and disinfecting the water sending channel when the endoscope cleaning and disinfecting device according to the first embodiment is used; Fig. 7 is a front view of an operation panel of the endoscope cleaning and disinfecting device according to the first embodiment; Fig. 8 is a schematic constitutional diagram of a control unit that controls the endoscope cleaning and disinfecting device according to the first embodiment; Fig. 9 is a flowchart explaining a cleaning and disinfecting operation of the endoscope to be performed by the endoscope cleaning and disinfecting device according to the first embodiment; Fig. 10 is a schematic constitutional diagram of an entire system of the endoscope cleaning and disinfecting device according to a second embodiment of the present invention; Fig. 11 is a schematic constitutional diagram of a state that the endoscope is connected with a channel plug, a cleaning tube for cleaning and disinfecting an air sending channel of the endoscope, and a cleaning tube for cleaning and disinfecting the water sending channel when the endoscope cleaning and disinfecting device according to the second embodiment is used; Fig. 12 is a schematic constitutional diagram of a state that the endoscope is connected with the channel plug, a biopsy valve cap, and a cleaning tube for cleaning and disinfecting a suction channel of the endoscope when the endoscope cleaning and disinfecting device according to the second embodiment is used; Fig. 13 is a schematic constitutional diagram of a main unit for a third embodiment of the present invention; Fig. 14 is a constitutional diagram of the main unit of the endoscope cleaning and disinfecting device according to a fourth embodiment of the present invention; Fig. 15 is a constitutional diagram of the endoscope cleaning and disinfecting device according to a fifth embodiment of the present invention; Fig. 16 is a constitutional diagram of a modified example of the fifth embodiment; Fig. 17 is a constitutional diagram of the endoscope cleaning and disinfecting device according to a seventh embodiment of the present invention; Fig. 18 is a constitutional diagram of a modified example of the seventh embodiment; Fig. 19 is a constitutional diagram of the endoscope cleaning and disinfecting device according to an eighth embodiment; Fig. 20 is a constitutional diagram of a modified example of the eighth embodiment; and Fig 21 is a constitutional diagram of another modified example of the eighth embodiment.

### BEST MODE FOR CARRYING OUT THE INVENTION

The present invention is described below in detail with reference to the accompanying drawings. The invention is not limited by embodiments below. Constitutional elements shown in the embodiments include ones that can be easily replaced by persons skilled in the art, or include ones substantially the same to the shown elements.

### (First Embodiment)

A first embodiment, which is not part of the present invention is explained below with reference to Figs. 1 to 9. Fig. 1 is a diagram of schematic constitutions of an endoscope cleaning and disinfecting device 1 and an endoscope 2 to be cleaned and disinfected according to the embodiment. Fig. 2 is a diagram of a schematic constitution of an entire system of an endoscope device 3 with which the endoscope 2 to be cleaned and disinfected by the endoscope cleaning and disinfecting device 1 according to the embodiment is incorporated.

The endoscope 2 has an elongate inserting unit 4 to be inserted into a coelom and an operating unit 5 on a user's side connected to a base end of the inserting unit 4. The operating unit 5 is connected with one end of a universal cord 6. The other end of the universal cord 6 is connected with a connector 7.

A front end constituting unit 8 is provided on a front end of the inserting unit 4. The front end constituting unit 8 contains a solid-state image sensing device of an observation optical system such as a CCD. A light guide cable (not shown in figures) is inserted into the operating unit 5 and the inserting unit 4. One end of the light guide cable is connected with an illumination optical system of the front end constituting unit 8, and the other end is connected with the connector 7 via the universal cord 6.

As shown in Fig. 2, the connector 7 is connected detachably with a light source device 9 of a peripheral apparatus. The connector 7 is further connected with one end of a signal cable 10. The other end of the signal cable 10 is connected with an image processing device 11. One end of the signal cable 10 is provided with a first connector 12, and the other end of the signal cable 10 is provided with a second connector 13. The first connector 12 of the signal cable 10 is connected detachably with the connector 7 of the endoscope 2, and the second connector 13 is connected detachably with the image processing device 11. Accordingly, a signal is transmitted and received between the endoscope 2 and the image processing device 11 via the signal cable 10.

The image processing device 11 is connected with an observation monitor 15 via a electric cable 14. An image signal from the image processing device 11 is supplied via the electric cable 14 to the observation monitor 15, so that an endoscopic image can be displayed.

Fig. 3 is a block diagram of the connecting state of the endoscope 2 and the image processing device 11 in the system of the endoscope device 3. The front end constituting unit 8 of the endoscope 2 is provided with an objective lens 16 and the CCD 17 of the observation optical system. The objective lens 16 forms an endoscopic image of a specimen on the CCD 17, and the endoscopic image of the specimen picked up by the CCD 17 is converted into an electric signal so as to be output to the connector 7.

A main unit of the endoscope 2 contains a rewritable nonvolatile memory 19 such as an EEPROM or a flash ROM, a CPU (Central Processing Unit) 20, and a reset circuit 21. The nonvolatile memory 19 is a memory that records and retains a plurality of data relating to the endoscope 2 such as a type, a model number, and channels information of the endoscope 2. The reset circuit 21 is an electric circuit that detects fluctuation and lowering of a power source voltage and outputs a reset signal.

The CPU 20 contains an operating circuit, a ROM (read-only memory), a RAM (random-access memory), a parallel communication port, a serial communication port, an A/D converter, and a counter. Programs that control external connecting devices such as the image processing device 11 and the cleaning and disinfecting device 1 are recorded onto the ROM in the CPU 20. The CPU 20 executes a process for writing and calling data into/from the memory 19, and a process for transmitting and receiving the data to/from the image processing device 11 and the cleaning and disinfecting device 1 according to the programs.

The image processing device 11 is provided with an endoscope connector 26, a monitor connector 27, and an operation panel 28 with which a user operates the image processing device 11. The endoscope connector 26 is connected detachably with the second connector 13 of the signal cable 10 on the side of the endoscope 2, so that the image processing device 11 is electrically connected with the endoscope 2.

The monitor connector 27 is connected with the electric cable 14 on the side of the monitor 15. The image processing device 11 is electrically connected with the observation monitor 15.

A main unit of the image processing device 11 contains a CCD driving power source 22, an image signal processing/changeover circuit 23, a CPU 24, and a rewritable nonvolatile memory 25 such as an EEPROM or a flash ROM. The CCD driving power source 22 is connected with the CCD 17 in the endoscope 2 via the signal cable 10, so that the CCD driving power source 22 drives the CCD 17.

The image signal processing/changeover circuit 23 is an electric circuit that executes an image process on an image signal photoelectrically converted by the CCD 17, and synthesizes/changes over a character signal such as including date and data of patient. The nonvolatile memory 25 is a memory that records and retains data transmitted from the endoscope 2.

The CPU 24 contains an operating circuit, a ROM, a RAM, a parallel communication port, a serial communication port, an A/D converter, and a counter. The CPU 24 is connected with the image signal processing/changeover circuit 23, the nonvolatile memory 25, the endoscope connector 26, and the operation panel 28.

The ROM in the CPU 24 records a program for controlling the image processing device 11. The CPU 24 controls the image processing device 11 according to the program. Accordingly, after the image signal processing/changeover circuit 23 processes an output signal transmitted from the CCD 17 of the endoscope 2 via the signal cable 10 to the image processing device 11, the image signal output from the image signal processing/changeover circuit 23 is input from the connector 27 via the electric cable 14 to the monitor 15 so that an endoscopic image obtained by the endoscope 2 is displayed on a screen of the monitor 15.

Table 1 shown below is an example of channels information in a plurality of data contents relating to the endoscope 2 stored in the internal memory 19 of the endoscope 2.

**(Table 1)**

| Name of scope type | Set value of flow rate in suction channel | Set value of flow rate in air/water sending channel | Set value of flow rate in forceps elevator wire channel |
|---|---|---|---|
| A | 2.0 L/min | 0.5 L/min | 0.1 L/min |
| B | 2.2 L/min | 0.8 L/min | No forceps elevator wire channel |
| C | 3.0 L/min | 1.2 L/min | No forceps elevator wire channel |
| ... | ... | ... | ... |
| ... | ... | ... | ... |

Numerical values shown in Table 1 are example values. The endoscope for duodenum whose scope type is A pre-stores data on set values of flow rate in the respective channels including, for example, 2.0 L/min as the set value of flow rate in the suction channel, 0.5 L/min as the set value of flow rate in the air/water sending channel, and 0.1 L/min as the set value of flow rate in the forceps elevator wire channel (these numerical values are temporary examples).

The main unit of the endoscope cleaning and disinfecting device 1 according to the embodiment is provided with a cleaning tab 29 in which a subject to be cleaned and disinfected such as the used endoscope 2 is placed. As shown in Fig. 4, the endoscope 2 to be cleaned and disinfected is placed in the cleaning tab 29.

An inner bottom portion of the cleaning tab 29 is provided with a circulation liquid suction port 30 and a drainage port 31. Further, an inner peripheral surface of the cleaning tab 29 is provided with a communication connector 32 (reading unit) for endoscope, a suction channel cleaning tube connection port 33, an air/water sending channel cleaning tube connection port 34, and a forceps elevator wire channel cleaning tube connection port 35, a feed water port 36, and a disinfectant injection port 37.

The communication connector 32 for endoscope is connected with the second connector 13 of the signal cable 10 of the endoscope 2 placed in the cleaning tab 29. When the communication connector 32 for endoscope is set to be connected with the second connector 13 of the signal cable 10, the endoscope 2 is electrically connected to the cleaning and disinfecting device 1, so that signals are sent and received between the endoscope 2 and the endoscope cleaning and disinfecting device 1.

The feed water port 36 is connected with one end of a feed water piping 38. The other end of the feed water piping 38 is connected with a tap 40 of a feed water source such as a water system or the like via a feed water valve 39.

The disinfectant injection port 37 is connected with one end of a feed disinfectant pump 41. The other end of the feed disinfectant pump 41 is connected with a bottom portion of a disinfectant tank 42 A feed disinfectant pump 43 is provided in the middle of the feed disinfectant pump 41.

The circulation liquid suction port 30 on the inner bottom portion of the cleaning tab 29 is connected with one end of a piping 44 for cleaning and disinfecting the insides of the endoscope channels. The other end of the piping 44 for cleaning and disinfecting the insides of the endoscope channels is branched into three channels, and the three branched piping 44a, 44b, and 44c are formed. The branched piping 44a is connected with the suction channel cleaning tube connection port 33, the branched piping 44b is connected with the air/water sending channel cleaning tube connection port 34, and the branched piping 44c is connected with the forceps elevator wire channel cleaning tube connection port 35.

A pump 45 for cleaning and disinfecting the insides of the endoscope channels, a check valve 46, and a flow rate sensor (measuring unit) 47 are sequentially provided in the middle of the piping 44 for cleaning and disinfecting the insides of the endoscope channels. The three branched piping 44a, 44b, and 44c are provided with cleaning and disinfecting channel switching electromagnetic valves 48a, 48b, and 48c, respectively.

A piping section 49 between the check valve 46 and the flow rate sensor 47 on the piping 44 is connected with an air supply piping 52 that is connected with a compressor 51 through a check valve 50.

The suction channel cleaning tube connection port 33 is connected with one end of a cleaning tube 53 (channel connector) for cleaning and disinfecting the suction channel of the endoscope 2, the air/water sending channel cleaning tube connection port 34 is connected with one end of a cleaning tube 54 (channel connector) for cleaning and disinfecting the air/water sending channel of the endoscope, and the forceps elevator wire channel cleaning tube connection port 35 is connected with one end of a cleaning tube 55 (channel connector) for cleaning and disinfecting the forceps elevator wire channel of the endoscope. The other end of the cleaning tube 54 for cleaning and disinfecting air/water sending channel is connected with an air/water sending channel (endoscope channel) 56 having a channel constitution shown in Fig. 5 of the endoscope 2 in the cleaning tab 29. The other end of the cleaning tube 53 for cleaning and disinfecting the suction channel is connected with a suction channel (endoscope channel) 57 having a channel constitution shown in Fig. 6 of the endoscope 2 in the cleaning tab 29, and the other end of the cleaning tube 55 for cleaning and disinfecting the forceps elevator wire channel is connected with the forceps elevator wire channel (not shown in figures) of the endoscope 2 in the cleaning tab 29.

The air/water sending channel 56 of the endoscope 2 has the channel constitution shown in Fig. 5, and the suction channel 57 has the channel constitution shown in Fig. 6. The front end constituting unit 8 of the inserting unit 4 of the endoscope 2 is provided with an air/water sending nozzle 58 shown in Fig. 5, and a front end opening 59a of a treatment tool through channel 59 which serves also as the suction channel that is provided in the inserting unit 4 of the endoscope 2 as shown in Fig. 6. Two air/water sending channels 60 and 61 (water sending channel 60 on the side of the inserting unit and an air sending channel 61 on the side of the inserting unit) are further provided in the inserting unit 4 of the endoscope 2. Front ends of the air/water sending channels 60 and 61 are connected with the air/water sending nozzle 58.

The operating unit 5 of the endoscope 2 on the user's side is provided with an air/water sending button cylinder 62, a suction cylinder 63, and a instrument channel port 64. A base end of the treatment tool through channel 59 is branched, and one channel 59b1 is connected with the instrument channel port 64, and the other channel 59b2 is connected with the suction cylinder 63. Base ends of the two air/water sending channels 60 and 61 are connected with a peripheral wall surface of the air/water sending button cylinder 62.

An outer peripheral surface of the connector 7 of the universal cord 6 is provided with two connectors 65a and 65b for air/water sending, and one suction connector 67. An inside of the universal cord 6 is provided with two air/water sending channels 68 and 69 (the water sending channel 68 on the side of the universal cord and the air sending channel 69 on the side of the universal cord) on the side of the universal cord, and one suction channel 70 on the side of the universal cord. One end of the air/water sending channel 68 is connected with the connector 65a, and one end of the air/water sending channel 69 is connected with the connector 65b. One connector 66 for sending air/water which is connected with the channel 69 on the side of the universal cord is protruded from a front end surface of the connector 7.

The other ends of the air/water sending channels 68 and 69 of the universal cord 6 are connected with the air/water sending button cylinder 62. The air/water sending channel 68 is connected with the inner bottom portion of the air/water sending button cylinder 62, and the air/water sending channel 69 is connected with the peripheral wall surface of the air/water sending button cylinder 62. A changeover operation of the air/water sending channel 56 shown in Fig. 5 is performed by operating an air/water sending channel changeover button (not shown) inserted into the air/water sending button cylinder 62.

One end of the suction channel 70 on the side of the universal cord is connected with the suction connector 67, and the other end of the suction channel 70 on the side of the universal cord is connected with the suction cylinder 63. A changeover operation of the suction channel 57 shown in Fig. 6 is performed by operating a suction changeover button (not shown) inserted into the suction cylinder 63.

An endoscope connector 71 of the cleaning tube 54 for cleaning and disinfecting the air/water sending channel is provided with, as shown in Fig. 5, an air/water sending cylinder inserting unit 72 inserted into the air/water sending button cylinder 62, and a suction cylinder inserting unit 73 inserted into the suction cylinder 63. A ring-shaped packing 74 is attached to a front end outer peripheral surface of the air/water sending cylinder inserting unit 72. The packing 74 pressure-contacts with the inner peripheral surface of the air/water sending button cylinder 62 of the endoscope 2 so as to separate the inside of the air/water sending button cylinder 62 into two spaces. In the state that the cleaning tube 54 is connected with the cylinder 62, the ring-shaped packing 74 keeps to connect the cleaning tube 54 with the air/water sending channel 68 in the cylinder 62 and simultaneously not to connect the cleaning tube 54 with the air/water sending channels 60, 61, and 69 connected with the cylinder 62.

When the air/water sending channel 56 shown in Fig. 5 is cleaned and disinfected, a bypass tube 75, as well as the cleaning tube 54 for cleaning and disinfecting the air/water sending channel, is used to connect the water sending channel 68 on the side of the universal cord and the air sending channel 69 on the side of the universal cord. One end of the bypass tube 75 is provided with a first cap connecting unit 76 to be connected with the two connectors 65a and 65b for sending air/water on the outer peripheral surface of the connector 7 of the universal cord 6, and the other end is provided with a second cap connecting unit 77 to be connected with a connector 66 for sending air/water on the front end surface of the connector 7.

When the air/water sending channel 56 of the endoscope 2 is cleaned and disinfected, as shown in Fig. 5, the endoscope connector 71 of the cleaning tube 54 for cleaning and disinfecting the air/water sending channel is attached to the operating unit 5 of the endoscope 2 on the user's side. Further, in the state that the bypass tube 75 is attached to the connector 7 of the universal cord 6, a cleaning liquid, a disinfectant or air is sent from the cleaning and disinfecting device 1 through the cleaning tube 54 for cleaning and disinfecting the water sending channel to the air/water sending channel 56 of the endoscope 2. At this time, when the cleaning liquid or the like is sent from the tube 54, as shown by arrows in Fig. 5, it is led to the water sending channel 68 on the side of the universal cord. The connector 7 of the universal cord 6 is connected with the bypass tube 75, and the bypass tube 75 connects the water sending channel 68 on the side of the universal cord with the air sending channel 69 on the side of the universal cord. For this reason, the cleaning liquid or the like that passes through the water sending channel 68 on the side of the universal cord is led to the air sending channel 69 on the side of the universal cord via the bypass tube 75 so as to return to the air/water sending button cylinder 62. The cleaning liquid or the like returned to the air/water sending button cylinder 62 is further led to the water sending channel 60 in the inserting unit and the air sending channel 61 in the inserting unit and passes through the inserting unit 4 of the endoscope 2 so as to emerge from an air/water sending nozzle 58 at the front end to the outside of the endoscope 2.

As the air, the cleaning liquid, the disinfectant, or the like are sent to the air/water sending channel 56 of the endoscope 2, a substantially single channel is constituted in appearance. Accordingly, a flow rate in the air/water sending channel 56 of the endoscope 2 can be detected accurately. That is to say, when the flow rate in the air/water sending channel 56 in the endoscope 2 reduces, an amount of the air actually sent to the endoscope 2 by the automatic cleaning and disinfecting device 1 becomes lower than a sending amount of the air pre-read from the endoscope 2. A flow rate of the air to be supplied to the endoscope 2 set in the automatic cleaning and disinfecting device 1 is measured as the amount of the air sent to the endoscope 2 by using the flow rate sensor 47 provided to the piping 44 for cleaning and disinfecting the insides of the endoscope channel. The device 1 compares the air sending amount with the set value of the air sending amount pre-read from the endoscope 2, thereby detecting a state in which the flow rate in the air/water sending channel 56 of the endoscope 2 is out of a range of the set value (a state in which the flow rate reduces).

An endoscope connector 78 of the cleaning tube 53 for cleaning and disinfecting the suction channel is provided with a cap connector 79 which is connected detachably with the instrument channel port 64 of the operating unit 5 of the endoscope 2 on the user's side. The cap connector 79 of the cleaning tube 53 for cleaning and disinfecting the suction channel is connected with the instrument channel port 64, so that the cleaning tube 53 for cleaning and disinfecting the suction channel is connected with the suction channel 57 of the endoscope 2 in the cleaning tab 29.

When the suction channel 57 of the endoscope 2 is cleaned and disinfected, as shown in Fig. 6, the cleaning tube 53 for cleaning and disinfecting the suction channel is connected with the suction channel 57 of the endoscope 2, and the endoscope connector 71 of the cleaning tube 54 for cleaning and disinfecting the water sending channel is attached to the operating unit 5 of the endoscope 2 on the user's side. In this state, the cleaning liquid, the disinfectant, the air, or the like is sent from the cleaning and disinfecting device 1 to the suction channel 57 of the endoscope 2 through the cleaning tube 53 for cleaning and disinfecting suction channel. At this time, the cleaning liquid or the like sent from the cleaning tube 53 for cleaning and disinfecting the suction channel to the instrument channel port 64 is branched from the branch channel 59b1 of the treatment tool through channel 59 at a connected portion between the channels 59b1 and 59b2 so as to be, as shown by an arrow in Fig. 6, split into a stream towards the front end opening 59a and a stream towards the suction cylinder 63. The stream of the cleaning liquid or the like towards the front end opening 59a flows out of the front end opening 59a.

Further, the opening of the suction cylinder 63 in the endoscope 2 is connected with the suction cylinder inserting unit 73 in the endoscope connector 71 of the cleaning tube 54 for cleaning and disinfecting the air/water sending channel so that the opening of the suction cylinder 63 is blocked. For this reason, the cleaning liquid or the like towards the suction cylinder 63 flows into the suction channel 70 on the side of the universal cord via the suction cylinder 63 so as to flow out of the suction connector 67 via the suction channel 70 on the side of the universal cord.

The air is sent from the cleaning tube 53 for cleaning and disinfecting the suction channel to the suction channel 57 of the endoscope 2. Its flow rate is measured and the measured flow rate is compared with the set value of the sending amount pre-read from the endoscope 2, thereby detecting the state that the flow rate in the suction channel 57 in the endoscope 2 is out of the range of the set value.

An endoscope connector 80 of the cleaning tube 55 for cleaning and disinfecting the forceps elevator wire channel is connected with the forceps elevator wire channel (not shown in figures) of the endoscope 2 in the cleaning tab 29. The forceps elevator wire channel (not shown in figures) is a single channel constitution having two openings at its inlet and its outlet. For this reason, when the forceps elevator wire channel is cleaned and disinfected, the cleaning liquid, the disinfectant, the air or the like is sent to the forceps elevator wire channel of the endoscope 2 via the cleaning tube 55 for cleaning and disinfecting the forceps elevator wire channel, in the state that the endoscope connector 80 of the cleaning tube 55 for cleaning and disinfecting the forceps elevator wire channel is connected with the forceps elevator wire channel (not shown in figures) of the endoscope 2 in the cleaning tab 29. At this time, when somewhere of the forceps elevator wire channel is clogged, the flow rate of the air sent from the cleaning tube 55 for cleaning and disinfecting the forceps elevator wire channel is measured, and the measured flow rate is compared with the set value of the sending amount. Accordingly, the state that the flow rate in the channel is out of the range of the set value can be easily detected.

Further, the drainage port 31 of the cleaning tab 29 is connected with an influent port 81a of a switching valve 81. The switching valve 81 has one influent port 81a and two effluent ports 81b and 81c. The effluent port 81b of the switching valve 81 is connected with one end of a recovery piping 82. The other end of the recovery piping 82 is connected with an upper portion of the disinfectant tank 42.

The other effluent port 81c of the switching valve 81 is connected with one end of a drainage piping 83. A drainage pump 84 is provided in the middle of the drainage piping 83.

The switching valve 81 switches a state among a state that the drainage port 31 is blocked and shut off, a state that the drainage port 31 is connected with the recovery piping 82, and a state that the drainage port 31 is connected with the drainage piping 83. The switching valve 81 is switched to the side of the drainage piping 83 and the drainage pump 84 is operated so that the liquid in the cleaning tab 29 is evacuated to out of the endoscope cleaning and disinfecting device 1.

Fig. 7 is a diagram of an operation panel 85 for user's operation of the endoscope cleaning and disinfecting device 1 according to the embodiment. The operation panel 85 has a program selecting button 86, a cleaning and disinfecting step start button 87, a stop button 88, a plurality of additional function buttons 89a to 89f, a cleaning duration display unit 90, a disinfecting duration display unit 91, a message code display unit (notifying unit) 92, and a disinfectant temperature display unit 93. The additional function buttons 89a to 89f has a function check button 89a, a leakage detecting button 89b, a alcohol flush button 89c, an air sending button 89d, a disinfectant warming button 89e, and a washing/rinsing button 89f, respectively.

When the operation panel 85 is operated, the cleaning duration and the disinfecting duration of the steps that are selected by the program selecting button 86 are displayed on the cleaning duration display unit 90 and the disinfecting duration display unit 91, respectively. Thereafter, the cleaning and disinfecting step start button 87 is pressed down, so that the steps are started.

Temperature of the disinfectant stored in the endoscope cleaning and disinfecting device 1 is displayed on the disinfectant temperature display unit 93. When the additional function buttons 89a to 89f are selected to be pressed down, besides the cleaning and disinfecting steps, the additional functions such as a step of measuring the flow rate in the internal channels and a disinfectant warming step of warming the disinfectant stored in the endoscope cleaning and disinfecting device 1 to set temperature can be executed.

The stop button 88 is pressed down so as to be capable of halting the cleaning and disinfecting step and the additional function steps. The endoscope cleaning and disinfecting device 1 of the present embodiment contains a notifying buzzer (notifying unit) 94 shown in Fig. 8 which notifies a user of a (operation) state that should be notified to the user. When information that should be notified to the user during the step is present besides information relating to the flow rate in the channels, the notifying buzzer 94 sounds, and a message code is displayed on a message code display unit 92. Accordingly, the (operation) state that should be notified to the user is notified. The message code displayed here is determined according to a message code table of Table 2.

**(Table 2)**

| Message code | Notifying contents |
|---|---|
| E01 | Water supply disrupted |
| E02 | Water discharged |
| ... | ... |
| ... | ... |
| ... | ... |
| E90 | Communication with endoscope is defective |
| E91 | Flow rate in endoscope channels decreases |
| E92 | Flow rate in endoscope channels increases |
| E93 | Flow rate is not detected |
| ... | ... |
| ... | ... |
| ... | ... |

Fig. 8 is a block diagram of a schematic constitution of a control unit 95 that controls the endoscope cleaning and disinfecting device 1 according to the embodiment. The control unit 95 has a CPU (control unit) 96 that controls equipment in the endoscope cleaning and disinfecting device 1. The CPU 96 contains a calculation circuit, a ROM, a RAM, a parallel communication port, a serial communication port, an A/D converter, and a counter (not shown in figures).

Further, the CPU 96 is connected with an analog circuit 97, a digital sensor 98, a driver 99, an operation panel 85, a reset circuit 100, and an endoscope communication connector 32, respectively. The analog circuit 97 is connected with an analog sensor 101 that monitors a state of the equipment in the endoscope cleaning and disinfecting device 1 and converts the monitored state into a serial electric signal. The analog circuit 97 amplifies an output from the analog sensor 101 so as to supply it to the CPU 96.

The digital sensor 98 is a sensor that monitors the state of the equipment in the endoscope cleaning and disinfecting device 1 and outputs a binarized electric signal to the CPU 96. The driver 99 is connected with the notifying buzzer 94 and a valve/pump 102 in the endoscope cleaning and disinfecting device 1. The notifying buzzer 94 and the valve/pump 102 in the endoscope cleaning and disinfecting device 1 are driven according to a control signal output from the driver 99.

The reset circuit 100 is an electric circuit that detects a fluctuation and a decrease in a power source voltage and outputs a reset signal. The ROM in the CPU 96 stores a program for controlling the equipment in the endoscope cleaning and disinfecting device 1. The CPU 96 executes the cleaning and disinfecting steps for the endoscope 2 according to the program.

A function of the endoscope cleaning and disinfecting device 1 having the above constitution according to the embodiment is explained below. After the check of the endoscope 2, in order to clean dirt of the used endoscope 2, the outer surface and the internal channels should be cleaned and disinfected every time of use.

In order to clean and disinfect the endoscope 2, the used endoscope 2 that is checked is set in the cleaning tab 29. At this time, the cleaning tubes 53, 54, and 55 are used so as to connect the channel ports of the air/water sending channel 56, the suction channel 57, and forceps elevator wire channel (not shown in figures) with the connection ports 33, 34, and 35 of the endoscope cleaning and disinfecting device 1. The endoscope 2 is connected with the bypass tube 75, so that the water sending channel 68 on the universal cord side is connected with the air sending channel 69 on the universal cord side in the endoscope 2. The communication cable 10 is used to electrically connect the endoscope 2 with the endoscope communication connector 32 of the endoscope cleaning and disinfecting device 1.

The contents of the cleaning and disinfecting steps are selected by the program selection button 86 on the operation panel 85 of the endoscope cleaning and disinfecting device 1, and the start button 87 for the cleaning and disinfecting steps is pressed down, so that the steps of cleaning, disinfecting, rinsing, and air sending are carried out according to the set conditions. At this time, the endoscope cleaning and disinfecting device 1 executes the cleaning and disinfecting steps for the endoscope 2 according to a flowchart shown in Fig. 9 as follows.

It is determined at step S1 whether the endoscope 2 set in the endoscope cleaning and disinfecting device 1 can communicate with the endoscope cleaning and disinfecting device 1. At this time, if the communication cable 10 is not connected, the communication is not properly made. When the CPU 96 of the endoscope cleaning and disinfecting device 1 cannot read the data stored in the endoscope 2, the sequence goes to next step S2. The notifying buzzer 94 in the endoscope cleaning and disinfecting device 1 sounds at step S2, and a message code E90 is displayed on the message code display unit 92. At this time, a message code "E90" is displayed on the message code display unit 92 of the operation panel 85 according to the contents of the message code table, so that the user is notified that the endoscope is not connected. The cleaning and disinfecting steps for the endoscope 2 are halted, and the user is supposed to check for the connection of the communication connector 32.

When the endoscope 2 communicates with the endoscope cleaning and disinfecting device 1 in a state that should not be notified to the user (when the condition which should be notified to the user does not occur) at step S1, the sequence goes to next step S3. The CPU 96 of the endoscope cleaning and disinfecting device 1 communicates with the CPU 20 in the endoscope 2 via the communication connector 32 for the endoscope and the communication cable 10 at step S3, and reads the data in the memory 19. The contents of the read data include a type, a model number, channel information, and the like of the endoscope 2. The CPU 96 of the endoscope cleaning and disinfecting device 1 recognizes the type of the endoscope 2 set in the cleaning tab 29 through the communication, and recognizes also data about the set values of the air sending amount in the air/water sending channel 56, the suction channel 57, and the forceps elevator wire channel (not shown in figures) of the endoscope 2. X is the set value of the air sending amount in the suction channel 57, Y is the set value of the air sending amount in the air/water sending channel 56, and Z is the set value of the air sending amount in the forceps elevator wire channel.

After the communication, the sequence goes to the cleaning step at step S4. At the cleaning step at step S4, the feed water valve 39 is opened, cleaning water is supplied from the tap 40 via the feed water piping 38 and the feed water port 36 into the cleaning tab 29. When water storage reaches a predetermined water level by the feed water into the cleaning tab 29, the feed water valve 39 is closed, so that the cleaning operation is started.

At the time of the cleaning operation, the cleaning and disinfecting channel switching electromagnetic valves 48a, 48b, and 48c, and the pump 45 for cleaning and disinfecting the insides of the endoscope channels cleaning and disinfecting device 1 are operated. According to the driving of the pump 45, the cleaning liquid in the cleaning tab 29 is sucked form the circulation liquid suction port 30 into the piping 44 for cleaning and disinfecting the insides of the endoscope channels. The cleaning liquid with high pressure to be discharged form the pump 45 is supplied from the piping 44 for cleaning and disinfecting the insides of the endoscope channels via the check valve 46, the flow rate sensor 47, and the three branched piping 44a, 44b, and 44c, into the cleaning tube 53 for cleaning and disinfecting the suction channel, the cleaning tube 45 for cleaning and disinfecting the air/water sending channel, and the cleaning tube 55 for cleaning and disinfecting the forceps elevator wire channel. The cleaning liquid is further supplied from these tubes 53, 54, and 55 into the air/water sending channel 56, the suction channel 57, and the forceps elevator wire channel (not shown in figures) in the endoscope 2, so that the endoscope channels are cleaned. That is to say, the cleaning water in the cleaning tab 29 is circulated through the piping 44 for cleaning and disinfecting the insides of the endoscope channels, so that channels in the endoscope 2 are cleaned.

When the cleaning operation is ended, the sequence goes to the rinsing step at step S5. At the rinsing step at step S5, the switching valve 81 is moved to a drainage position, and simultaneously the drainage pump 84 is driven. According to the driving of the drainage pump 84, the cleaning liquid in the cleaning tab 29 is sucked from the drainage port 31 via the switching valve 81 to the drainage piping 83, so as to be drained to the outside through the drainage piping 83.

An operation of the compressor 51 is started at the latter half of the rinsing step. High-pressure air injected form the compressor 51 passes through the air supply piping 52 and is supplied to the cleaning tubes 53, 54, and 55 via the check valve 50, the flow rate sensor 47, and the three branched piping 44a, 44b, and 44c. The air is supplied into the channels in the endoscope 2 via the cleaning tubes 53, 54, and 55, and the channels in the endoscope 2 are dried.

During the channel draining operation, the flow rate of air in the channels of the endoscope 2 is measured at next step S6. At the measurement of the flow rate at step S6, the cleaning and disinfecting channel switching electromagnetic valves 48a, 48b, and 48c are operated so as to send air individually to the channels. Outputs from the flow rate sensor 47 are read for the respective channels, so that the flow rates of the air in air/water sending channel 56, the suction channel 57, and the forceps elevator wire channel (not shown in figures) are measured.

For example, when the air is sent in a state that the electromagnetic valve 48a is opened and the electromagnetic valves 48b and 48c are closed, the air is sent only to the suction channel 57 of the endoscope 2. The output from the flow rate sensor 47 at this time is read, so that the air sending flow rate in the suction channel 57 of the endoscope 2 is measured.

Subsequently, when the air is sent in a state that the electromagnetic valve 48b is opened and the electromagnetic valves 48a and 48c are closed, the air is sent only to the air/water sending channel 56 in the endoscope 2. The output from the flow rate sensor 47 at this time is read so that the air sending flow rate in the air/water sending channel 56 of the endoscope 2 is measured.

Further, when the air is sent in a state that the electromagnetic valve 48c is opened and the electromagnetic valves 48a and 48b are closed, the air is sent only to the forceps elevator wire channel in the endoscope 2. The output from the flow rate sensor 47 at this time is read so that the air sending flow rate in the forceps elevator wire channel of the endoscope is measured.

"x" here is a measured value of the air flow rate in the suction channel 57, "y" is a measured value of the air flow rate in the air/water sending channel 56, and "z" is a measured value of the air flow rate in the forceps elevator wire channel.

After the air flow rate in the channels of the endoscope 2 is measured, the operation process at step S7 is executed. The measured data (x, y, z) of the air flow rate in the channels of the endoscope 2 measured by the flow rate sensor 47 are compared with the set values (X, Y, Z) of the air sending amount in the channels of the endoscope obtained in advance by communication with the endoscope 2. A determination is made whether the flow rates in the channels are within the range of the set values (whether the air and the liquid are sent sufficiently to the air/water sending channel 56, the suction channel 57, and the forceps elevator wire channel in the endoscope) or is out of the range of the set values.

In the comparison determining method, the ratio of the measured values to the set values is calculated according to the following expression, and when the ratio as the operation result is within a certain set range, a (operation) state is such that it is not necessary to notify the user. When the ratio is out of the range, a (operation) state is such that the user should be notified.

Expression: (actually measured air sending flow rate)÷(air sending flow rate when the flow rate is within the range of the set values)x100, wherein A is a ratio of the set value X of the air sending amount in the suction channel 57 to the measured value x of the air flow rate calculated at this time, B is a ratio of the set value Y of the air sending amount in the air/water sending channel 56 to the measured value y of the air flow rate, and C is a ratio of the measured value Z of the air sending amount in the forceps elevator wire channel to the measured value z of the air flow rate.

The range of the set values of the flow rates in the channels in the ratio as the operated result is set to, for example, 80% to 120%. When the set value of the air sending amount in the suction channel 57 of the endoscope 2 obtained by the communication is, for example, 2.3 L/min, and the measured air sending amount is, for example, 2.0 L/min, the ratio of the operated result is 87%. Since the flow rate in the channel is within the range of the set value, a determination is made that the (operation) state is such that it is not necessary to notify the user.

For example, when the set value of the air sending amount in the channel is 2.3 L/min and the measured air sending amount is 1.2 L/min, the ratio A of the calculation result is about 53%. In this case, the ratio A of the calculation result deviates downward from the set range of the calculation state that does not have to be notified to the user. For this reason, a determination is made that the channel is clogged and the compressor 51 does not operate properly, or the air sending to the endoscope channels is insufficient because, for example, the cleaning tubes 53, 54, and 55 are forgotten to be connected. That is to say, the determination is made as the (operation) state which should be notified to the user.

Further, when the set value of the air sending amount in the channel is 2.3 L/min and the measured air sending amount is 5.0 L/min, the calculation result is about 217%. In this case, since the calculation result deviates upward from the range of the set value, a determination is made that the cleaning tubes 53, 54, and 55 are disconnected, the channels in the cleaning and disinfecting device 1 are disconnected, or a leakage occurs. The determination is made as the (operation) state which should be notified to the user.

The set values of the air sending amount in the channels of the endoscope are compared with the measured air sending amount in the channels of the endoscope. When a determination is made that the flow rate in even one of the channels in the endoscope 2 is out of the range of the set value, the cleaning and disinfecting steps are halted, and the (operation) state is notified to the user.

That is to say, a determination is made at step S8 whether the ratios (A, B, and C) of the calculation result in the channels of the endoscope 2 are larger than 80% as a lower limit value of the set range in the (operation) state which does not have to be notified to the user (A>80%, B>80%, and C>80%). When the ratios (A, B, C) of the calculation result are smaller than 80% as the lower limit value of the set range in the state which does not have to be notified to the user at step S8 (the (operation) state which should be notified to the user is generated), the sequence goes to next step S9. The notifying buzzer 94 in the endoscope cleaning and disinfecting device 1 is sounded at step S9, and a message code "E91" which represents the state that the calculation result deviates downward from the range of the (operation) state which does not have to be notified to the user is displayed on the message code display unit 92 of the operation panel 85 according to the message code table of Table 2. Accordingly, the occurrence of the (operation) state which should be notified to the user and its contents are notified.

When the ratios (A, B, and C) of the calculation result are larger than 80 as the lower limit of the set range of the (operation) state which does not have to be notified to the user (the (operation) state which should be notified to the user is not occurred) at step S8, the sequence goes to next step S10. A determination is made at step S10 whether the ratios (A, B, and C) of the calculation result for the channels of the endoscope 2 is smaller than 120% as the upper limit value of the set value in the (operation) state which does not have to be notified to the user (A<120%, B<120%, and C<120%). When the ratios (A, B, and C) of the calculation result are larger than 120% as the upper limit value of the set range in the (operation) state which does not have to be notified to the user (when the (operation) state which should be notified to the user is occurred), the sequence goes to next step 511. The notifying buzzer 94 in the endoscope cleaning and disinfecting device 1 is sounded at step S11, and a message code "E92" which represents that the calculation results deviates upward from the range of the (operation) state which does not have to be notified to the user is displayed on the message code display unit 92 of the operation panel 85 according to the message code table of Table 2. Accordingly, the occurrence of the (operation) state which should be notified to the user and its contents are notified.

When the (operation) state is determined that the amount of the air sent to all the endoscope channels does not have to be notified to the user, a determination is made that the endoscope channels are cleaned sufficiently. The sequence goes to the disinfecting step at next step S12.

When the (operation) state which should be notified to the user is not present, the disinfecting step is started. At the disinfecting step, the disinfectant in the disinfectant tank 42 is first injected into the cleaning tab 29 from the disinfectant injection port 37 through the feed disinfectant pump 41 by the operation of the feed disinfectant pump 43. When a storage amount of the disinfectant in the cleaning tab 29 reaches the predetermined water level, the entire endoscope 2 is soaked completely in the disinfectant. The outer surface of the endoscope 2 is disinfected by the soaking in the disinfectant.

At the same time, the pump 45 for cleaning and disinfecting insides of endoscope channels is operated so as to suck the disinfectant in the cleaning tab 29 and feed the liquid into the channels in the endoscope 2 through the cleaning tubes 53, 54, and 55. Accordingly, the channels are disinfected. In such a manner, the outer surface of the endoscope 2 and the insides of the channels are disinfected.

After the disinfecting step is started, when predetermined disinfecting duration passes, the operation of the pump 45 for cleaning and disinfecting endoscope channels is stopped, and the switching valve 81 switches to a disinfectant recovery position. In this state, the disinfectant tank 42 is connected with the drainage port 31 of the cleaning tab 29, so that the disinfectant is drained from the cleaning tab 29 and recovered into the disinfectant tank 42.

Similarly to the operation at the cleaning step, the rinsing step at step S13 is executed and the compressor 51 is operated at the latter half of the step. Air is supplied into the channels of the endoscope 2 so that the drying and air sending step at step S14 is executed. The endoscope 2 is cleaned and disinfected by the above steps.

The above constitution produces the following effects. That is to say, in the endoscope cleaning and disinfecting device 1 according to the embodiment, the CPU 96 of the endoscope cleaning and disinfecting device 1 reads the data stored in the endoscope 2 in a state that the used endoscope 2 is set in the cleaning tab 29 at the time of the cleaning and disinfecting operation for the endoscope 2. During the cleaning and disinfecting operation for the endoscope 2, the flow rates of the air in the channels of the endoscope 2 are measured. After the measurement, the measured data (x, y, and z) of the air flow rates in the channels of the endoscope measured by the flow rate sensor 47 are compared with the set values (X, Y, and Z) of the air sending amount of the endoscope 2 obtained by the communication with the endoscope 2. The operation process for determining whether the flow rates are within the range of the set values is executed. When the flow rate in even one of the channels in the endoscope 2 is out of the range of the set value, the cleaning and disinfecting steps are halted, and the notifying buzzer 94 in the endoscope cleaning and disinfecting device 1 is sounded. A message code is displayed on the message code display unit 92 of the operation panel 85, so that the occurrence of the (operation) state which should be notified to the user and its contents are notified. For this reason, in the endoscope cleaning and disinfecting device 1 according to the embodiment, various states of the channels in the endoscope 2, the (operation) state of the compressor 51, the pumps and the like in the endoscope cleaning and disinfecting device 1 which should be notified to the user, disconnection of the cleaning tubes 53, 54, and 55, and the like can be detected accurately with a simple constitution. Accordingly, the endoscope 2 can be cleaned and disinfected suitably.

The state that the flow rates in the channels of the endoscope are out of the range of the set values due to air, the (operation) state of the compressor 51 in the endoscope cleaning and disinfecting device 1 which should be notified to the user, and the disconnection of the cleaning tubes 53, 54, and 55 are detected. Further, the flow rate of the cleaning water or the disinfectant flowing through the piping 44 for cleaning and disinfecting insides of endoscope channels is measured. The flow rate of the channel which is within the range of the set value is prestored in the CPU 20 of the endoscope 2, and they may be compared with each other at the time of the endoscope cleaning and disinfecting steps. In this case, the (operation) states of the piping 44 for cleaning and disinfecting insides of endoscope channels and the pump 45 for cleaning and disinfecting insides of endoscope channels which should be notified to the user can be also detected.

In the embodiment, the flow rate sensor 47 measures the flow rates, but instead of the flow rate sensor 47, a pressure gage may be used to measure a pressure, and the (operation) state which should be notified to the user may be detected by using the pressure data.

In the embodiment, the flow rates are measured when the cleaning step is ended, and detection is made whether the flow rates in the channels are out of the range of the set values. After the cleaning and disinfecting steps are started, however, immediately the air is sent into the endoscope channels, and a determination may be obviously made whether the flow rates in the channels are out of the range of the set values. Further, this measurement may be made as an additional function, independently as "a check for reduction in the flow rates in the endoscope channels".

In the embodiment, when the endoscope 2 set in the cleaning tab 29 cannot communicate with the endoscope cleaning and disinfecting device 1, the cleaning and disinfecting steps are halted, and this is notified to the user. The cleaning and disinfecting steps may not be, however, halted, and special cleaning and disinfecting steps for the case that the preset flow rates are out of the range of the set values may be executed. At the time when the special cleaning and disinfecting steps are ended, it may be notified to the user that the reduction in the flow rates in the channels of the endoscope is not checked.

As this notifying method, a message code E93 may be displayed on the message code display unit 92 of the operation panel 85 according to the contents of the message code table. In another method, the operation panel 85 may have an LED, a lamp or the like which indicates that the detection of the reduction in the flow rates in the channels is unconfirmed, and they may be turned on.

On the contrary, needless to say, the operation panel 85 may have an LED, a lamp, or the like which indicates that the detection of the reduction in the flow rates in the channels is confirmed. When the flow rates in the channels do not decrease, the LED or the lamp is turned on, and when the reduction in the flow rates in the channels is not detected, the LED or the lamp is turned off.

In the embodiment, when the (operation) state which is predicted to be caused by deviation of the flow rates in the channels of the endoscope from the set values and should be notified to the user, is detected, the cleaning and disinfecting steps are immediately halted, and the state is notified to the user. The cleaning step is added and the state that the flow rates deviate from the set values is tried to be removed, and after the additional cleaning step, the reduction in the flow rates in the channels is again detected. In such a manner, a check may be made whether the state that the flow rates deviate from the range of the set values is removed. At this time, at the additional cleaning step, a liquid sending pressure of the cleaning water to the channels may be raised, or a special program may be carried out by a gas-liquid two-phase system in order that the flow rates fall within the range of the set values.
After the additional cleaning step, when the flow rates can be within the range of the set values, the steps after the cleaning step are executed, and when the flow rates cannot be within the range of the set values, the (operation) state which should be notified to the user is notified.

Even when the state that the flow rates in the channels of the endoscope deviate from the set values is found, the planned step is continued, and after the cleaning and disinfecting steps are completed, the (operation) state which should be notified to the user may be notified. In this case, the outer surface and the channel whose flow rate does not deviate from the set value are cleaned and disinfected.

In the embodiment, the information about the endoscope channels is checked by connecting the endoscope 2 and the endoscope cleaning and disinfecting device 1 using the communication cable 10. However, the present invention is not limited to this, and various systems such as communication using infrared radiation and magnetism can also be considered without departing from the spirit of the invention.

The information about the channels of respective types of endoscopes is pre-stored in the endoscope cleaning and disinfecting device 1, and the user can input or select a type of an endoscope on the panel of the endoscope cleaning and disinfecting device 1.

In the embodiment, the (operation) state which should be notified to the user is notified by displaying a message code, but a liquid crystal panel, a monitor, a printer, or the like is provided to the endoscope cleaning and disinfecting device 1, so that error contents and a cause of the (operation) state which should be notified to the user, a countermeasure, and the like may be displayed or printed in a form of a sentence. Further, also when the planned cleaning and disinfecting steps are carried out without notifying the (operation) state to the user, needless to say, a name and a model number of the cleaned/disinfected endoscope 2, contents of the cleaning and disinfecting steps, date and time, a checked result of the detection of the state that the flow rates deviate from the set values, and the execution of the cleaning and disinfecting steps without notifying the (operation) state to the user may be displayed or printed.

### (Second Embodiment)

Figs. 10 to 12 are diagrams of a second embodiment, which is not part of the present invention. In the second embodiment, the constitution of the endoscope cleaning and disinfecting device 1 according to the first embodiment (see Figs. 1 to 9) is modified as follows. In Figs. 10 to 12, the same components in the second embodiment as those in the first embodiment are designated by the same reference signs, and explanations thereof are omitted.

That is to say, in the second embodiment, the connecting positions of the cleaning tubes 53, 54, and 55 to be connected to the endoscope 2 set in the cleaning tab 29 of the endoscope cleaning and disinfecting device 1 are arranged on the connector 7 on the side of the universal code 6 in the endoscope 2 as shown in Fig. 10.

In Fig. 10, an inner peripheral surface of the cleaning tab 29 of the endoscope cleaning and disinfecting device 1 is provided with the communication connector 32 for endoscope, the suction channel cleaning tube connection port 33, and the forceps elevator wire channel cleaning tube connection port 35 similarly to the first embodiment. Further, the inner peripheral surface is provided with an air sending channel cleaning tube connection port 111 and a water sending channel cleaning tube connection port 112.

The four branched piping 44a, 44b, 44c, and 44d are formed on a lower stream (branched piping) side of the piping 44 for cleaning and disinfecting insides of endoscope channels provided in the main unit of the endoscope cleaning and disinfecting device 1. The branched piping 44a is connected with the suction channel cleaning tube connection port 33, and the branched piping 44b is connected with the air sending channel cleaning tube connection port 111. The branched piping 44c is connected with the water sending channel cleaning tube connection port 112, and the branched piping 44d is connected with the forceps elevator wire channel cleaning tube connection port 35.

The cleaning and disinfecting channel switching electromagnetic valves 48a, 48b, 48c, and 48d are provided in the middle of the branched piping 44a, 44b, 44c, and 44d of the piping 44 for cleaning and disinfecting insides of endoscope channels, respectively.

The inside of the cleaning tab 29 is provided with four cleaning tubes 113 to 116 for cleaning and disinfecting the endoscope channels. One end of the cleaning tube 113 is connected with the suction channel cleaning tube connection port 33, so that a cleaning tube for cleaning and disinfecting the channels of the endoscope 2 is formed. The other end of the cleaning tube 113 for cleaning and disinfecting the suction channel is provided with a suction connector 117. As shown in Fig. 12, the suction connector 117 of the cleaning tube 113 for cleaning and disinfecting the suction channel is connected detachably with the suction connector 67 of the connector 7 of the endoscope 2 set in the cleaning tab 29.

One end of the cleaning tube 114 is connected with the air sending channel cleaning tube connection port 111, so that a cleaning tube for cleaning and disinfecting the air sending channel is formed. The other end of the cleaning tube 114 for cleaning and disinfecting the air sending channel is provided with two branched tubes 114a and 114b which are branched as shown in Fig. 11. A front end of the branched tube 114a is provided with a first air sending connector 118a. Further, a front end of the branched tube 114b is provided with a second air sending connector 118b. The first air sending connector 118a of the branched tube 114a of the cleaning tube 114 for cleaning and disinfecting the air sending channel is connected detachably with the connector 65b on the outer peripheral surface of the connector 7 of the endoscope 2. The second air sending connector 118b of the branched tube 114b of the cleaning tube for cleaning and disinfecting the air sending channel is connected detachably with the connector 66 on the front end surface of the connector 7 of the endoscope 2.

One end of the cleaning tube 115 is connected with the water sending channel cleaning tube connection port 112, so that a cleaning tube for cleaning and disinfecting the water sending channel is formed. The other end of the cleaning tube 115 for cleaning and disinfecting the water sending channel is provided with a water sending connector 119 as shown in Fig. 11. The water sending connector 119 of the cleaning tube 115 for cleaning and disinfecting the water sending channel is connected detachably with the water sending connector 65a of the connector 7 of the endoscope 2 set in the cleaning tab 29.

One end of the cleaning tube 116 is connected with the forceps elevator wire channel cleaning tube connection port 35, so that a cleaning tube for cleaning and disinfecting the forceps elevator wire channel is formed. The other end of the cleaning tube 116 for cleaning and disinfecting the forceps elevator wire channel is provided with an endoscope connector 120 of the cleaning tube 116. The endoscope connector 120 of the cleaning tube 116 for cleaning and disinfecting the forceps elevator wire channel is connected with the forceps elevator wire channel (not shown) of the endoscope 2 in the cleaning tab 29.

A cylinder cap connector 121 which blocks off the air/water sending button cylinder 62 and the suction cylinder 63, and a forceps cap connector 122 which blocks off the instrument channel port 64 are attached to the operating unit 5 of the endoscope 2 set in the cleaning tab 29. Further, cylinder cap connector 121 is provided with an air/water sending cylinder inserting unit 123 which is inserted into the air/water sending button cylinder 62, and a suction cylinder inserting unit 124 which is inserted into the suction cylinder 63. A ring-shaped packing 125 is attached to an outer peripheral surface of the air/water sending cylinder inserting unit 123. The packing 125 pressure-contacts with the inner peripheral surface of the air/water sending button cylinder 62 of the endoscope so as to divide the inside of the air/water sending button cylinder 62 into two spaces. The two spaces include an air sending channel 126 and a water sending channel 127.

A function of the endoscope cleaning and disinfecting device 1 having the above constitution according to the embodiment is explained below. When the endoscope 2 is cleaned and disinfected by the endoscope cleaning and disinfecting device 1 according to the embodiment, the used endoscope 2 which is used for a check is set in the cleaning tab 29. At this time, the suction connector 117 of the cleaning tube 113 for cleaning and disinfecting channels is connected with the suction connector 67 of the connector 7 of the endoscope 2. Further, the first air sending connector 118a of the cleaning tube 114 for cleaning and disinfecting air sending channel is connected with the connector 65b on the outer peripheral surface of the connector 7 of the endoscope 2, and the second air sending connector 118b is connected with the connector 66 on the front end surface of the connector 7 of the endoscope 2. The water sending connector 119 of the cleaning tube 115 for cleaning and disinfecting water sending channel is connected with the water sending connector 65a of the connector 7 of the endoscope 2, and the endoscope connector 120 of the cleaning tube 116 for cleaning and disinfecting forceps elevator wire channel is connected with the forceps elevator wire channel (not shown) of the endoscope 2. The cylinder cap connector 121 which blocks off the air/water sending button cylinder 62 and the suction cylinder 63, and the forceps cap connector 122 which blocks off the instrument channel port 64 are attached to the operating unit 5 of the endoscope 2.

Accordingly, an air/water sending system for cleaning and disinfecting air/liquid sending channel shown in Fig. 11 is constituted, and an air/liquid sending system for cleaning and disinfecting the suction channel shown in Fig 12 is constituted. The cleaning liquid and the air fed from the cleaning tube 115 for cleaning and disinfecting the water sending channel pass from the connector 7 of the endoscope 2 through the water sending channel 68 on the side of the universal cord so as to reach the air/water sending button cylinder 62 of the operating unit 5 as shown by an arrow in Fig. 11. Further, the cleaning liquid and the air are led to the water sending channel 60 on the side of the inserting unit, and come out of the endoscope 2 from the air/water sending nozzle 58 at the front end of the inserting unit 4.

The cleaning liquid and the air fed from the cleaning tube 114 for cleaning and disinfecting the air sending channel pass from the connector 7 of the endoscope 2 through the air sending channel 69 on the side of the universal cord so as to reach the air/water sending button cylinder 62 of the operating unit 5 as shown by an arrow of Fig. 11. The cleaning liquid and the air are further led to the air sending channel 61 on the side of the inserting unit so as to come out of the endoscope 2 from the air/water sending nozzle 58 on the front end of the inserting unit 4.

The cleaning liquid and the air fed from the cleaning tube 113 for cleaning and disinfecting the suction channel pass from the connector 7 of the endoscope 2 through the suction channel 70 on the side of the universal cord so as to reach the suction channel button cylinder 63 of the operating unit 5 as shown by an arrow of Fig. 12. Since the opening of the cylinder 63 is blocked by the cylinder cap connector 121, the cleaning liquid and the air are further led to the treatment tool through channel 59 as the suction channel on the side of the inserting unit. Since the instrument channel port 64 in the middle of the treatment tool through channel 59 is also blocked by the forceps cap connector 122, the cleaning liquid and the air are led to the front end of the inserting unit 4 so as to come out of the endoscope 2.

According to the embodiment, since the air sending channel 126 and the water sending channel 127 in the endoscope 2 are constituted like one channel respectively, when the flow rate decreases in a certain place of the channels, the flow rate greatly changes. For this reason, similarly to the first embodiment, before the cleaning and disinfecting steps, the endoscope 2 is electrically connected and communicated with the endoscope communication connector 32 i n the endoscope cleaning and disinfecting device 1 by the communication cable 10. Accordingly, the information about the channels are obtained from the endoscope 2, and the flow rate during the actual cleaning and disinfecting steps is detected so that both of them are compared with each other. Accordingly, the (operation) state such as a reduction in the flow rates in the channels which should be notified to the user can be detected.

According to the embodiment, since the suction channel 57 in the endoscope 2 is also constituted like one channel, when the flow rate reduces in a certain place of the channel, the flow rate in the channel changes more greatly than the case where the flow rate is within the range of the set value. For this reason, similarly to the first embodiment, the (operation) state which should be notified to the user can be detected easily.

### (Third Embodiment)

Fig. 13 is a diagram of a third embodiment, which is not part of the present invention. In the third embodiment, the constitution of the endoscope cleaning and disinfecting device 1 according to the second embodiment (see Figs. 10 to 12) is modified as follows.

That is to say, in the second embodiment, in order to clean the air/water sending channel in the endoscope 2 set in the cleaning tab 29 of the endoscope cleaning and disinfecting device 1, the cleaning tube 114 for cleaning and disinfecting the air sending channel, and the cleaning tube 115 for cleaning and disinfecting the water sending channel are provided. The two cleaning tubes 114 and 115 are connected with the connector 7 on the side the universal cord 6 in the endoscope 2, so that the air/water sending channel of the endoscope 2 set in the cleaning tab 29 is divided into the air sending channel 126 and the water sending channel 127. Accordingly, the air and the water are sent into the air/water sending channel in the endoscope 2. In the third embodiment, however, as shown in Fig. 13, the cleaning tube 114 for cleaning and disinfecting the air sending channel and the cleaning tube 115 for cleaning and disinfecting the water sending channel in the second embodiment are integrated as one cleaning tube 131 for cleaning and disinfecting the air/water sending channel. The air and the water are sent simultaneously from one cleaning tube 131 into the air/water sending channel of the endoscope 2.

Further, two branched tubes 131a and 131b are provided on the endoscope connector of the cleaning tube 131. A front end of the branched tube 131a is provided with a first air/liquid sending piping connector 132a. The first air/liquid sending piping connector 132a is provided with two concave portions 133a and 133b which are connected detachably with the connectors 65a and 65b on the outer peripheral surface of the connector 7 of the endoscope 2.

A front end of the branched tube 131b is provided with a second air/liquid sending piping connector 132b. The second air/liquid piping connector 132b is provided with a concave portion 134 which is connected detachably with the connector 66 on the front end surface of the connector 7 of the endoscope 2.

A cylinder cap connector 135 is attached to the air/water sending button cylinder 62 and the suction cylinder 63 of the endoscope 2. The cylinder cap connector 135 is provided with an air/water sending button cylinder blocking unit 135a which blocks off the opening of the air/water sending button cylinder 62, and a suction cylinder blocking unit 135b which blocks off the opening of the suction cylinder 63.

The method of sending the air and the liquid from the endoscope cleaning and disinfecting device 1 into the suction channel 57 and the forceps elevator wire channel of the endoscope 2 has like constitution as that in the second embodiment. Therefore, explanations thereof are omitted.

When the endoscope cleaning and disinfecting device 1 according to the third embodiment is used, the first air/liquid sending piping connector 132a of the branched tube 131a of the cleaning tube 131 for cleaning and disinfecting the air/water sending channel is connected detachably with the connectors 65a and 65b on the outer peripheral surface of the connector 7 of the endoscope 2. The second air/liquid sending piping connector 132b of the branched tube 131b of the cleaning tube 131 is connected detachably with the connector 66 on the front end surface of the connector 7. In this state, the air and the cleaning liquid fed from the endoscope cleaning and disinfecting device 1 through the cleaning tube 131 for cleaning and disinfecting the air/water sending channel reach the air/water sending button cylinder 62 of the operating unit 5 from the connector 7 of the endoscope 2 via the water sending channel 68 and the air sending channel 69 on the side of the universal cord. Since the opening of the cylinder 62 is blocked by the cylinder cap connector 135, the fed air, the cleaning liquid, and the like are further led to the water sending channel 60 and the air sending channel 61 on the side of the inserting unit, so as to come out of the endoscope 2 from the air/water sending nozzle 58 on the front end of the inserting unit 4.

The above constitution produces the following effect. That is to say, according to the embodiment, when the flow rates decrease in a certain place in the air sending channel and the water sending channel of the endoscope 2, the flow rates in the air sending channel and the water sending channel of the endoscope 2 decrease. For this reason, the (operation) state which should be notified to the user can be detected. For this reason, in the embodiment, in order to clean the air/water sending channel of the endoscope 2, a number of the cleaning tubes 131 to be connected with the connector 7 of the endoscope 2 can be decreased by one in comparison with the second embodiment. For this reason, a number of parts, the control method, and the like in the endoscope cleaning and disinfecting device 1 can be further simplified in comparison with the second embodiment.

### (Fourth Embodiment)

In the endoscope cleaning and disinfecting device 1 according to the first embodiment, when a clogged state of the endoscope channels is detected, the air is supplied from the compressor 51 to all of the suction channel 56, the air/water sending channel 57, and the forceps elevator wire channel (not shown) with equal pressure (see Fig. 4). Since the forceps elevator wire channel, however, has a small diameter and a wire for rising the forceps therein, resistance in the channel is remarkably higher than the other endoscope channels. For this reason, since the flow rate of the passing air in the forceps elevator wire channel is remarkably small, it is difficult to measure the flow rate (S6) using the flow rate sensor 47. The fourth embodiment, therefore, has such a characteristic that the supply pressure of the air is regulated according to types of the endoscope channels, and the sufficient flow rate is secured for the measurement of the flow rates (S6).

Fig. 14 is a constitutional diagram of a main unit of the endoscope cleaning and disinfecting device according to an embodiment of the present invention. In Fig. 14, like components as those in the first embodiment are designated by like reference signs, and explanations thereof are omitted. The endoscope cleaning and disinfecting device 140 is different from the endoscope cleaning and disinfecting device 1 according to the first embodiment in that an air/water sending system 141 which supplies a fluid to the suction channel 56 and the like includes relief valves 142a to 142c, and a relief piping 143. The relief valves 142a to 142c are provided on upper stream sides of the electromagnetic valves 48a to 48c on the branched piping 44a to 44c, respectively. The relief valves 142a to 142c relieve a part of the passing air to the relief piping 143, so that the pressure of the air supplied into the endoscope channels is decreased to a predetermined set value. Accordingly, the air whose pressure is regulated to the predetermined one is supplied into the endoscope channels. The pressure of the air is set in a manner that springs provided to the relief valves 142a to 142c are manually adjusted individually. The set values are known values which are specific to the respective endoscope channels. The set values are read by the CPU 96 of the control unit, and the set values are compared with the actually measured values (see S6 to S8) so that the clogging state of the endoscope channels is detected. The relief piping 143 is drawn out from the relief valves 142a to 142c, and is connected with the upper stream side of the pump 45. The compressor 144 can supply air with pressure at least equal to ore more than resistance pressure of the endoscope channels.

In the fourth embodiment, the clogged state in the endoscope channels is detected similarly to the first embodiment (see Fig. 9). That is to say, the compressor 144 is driven at the end of the rinsing step (S5), and the electromagnetic valves 48a to 48c are sequentially switched so that the air can be supplied to the endoscope channels. The insides of the endoscope channels are dried by using the air, and simultaneously the flow rate sensor 47 measures the flow rates of the air so as to detect presence of the clogging in the endoscope channels (S6 to S11). The pressure of the air to be supplied to the endoscope channels becomes higher in the endoscope channels where resistance of the endoscope channels is larger and it is more difficult for the fluid to flow. Specifically, the air is supplied to the forceps elevator wire channel where the resistance is the largest with higher pressure in comparison with the other endoscope channels 56 and 57. As a result, since the air can be supplied to the forceps elevator wire channel where it is difficult for the fluid to flow with enough pressure to measure the flow rates, the measurement accuracy of the flow rate sensor 47 can be improved.

In the fourth embodiment, the pressure of the air passing through the relief valves 142a to 142c is equal to or less than the resistance pressure of the endoscope channels. For example, the resistance pressure is about 0.3 [MPa] in the suction channel 56, about 0.2 [MPa] in the air/water sending channel 57, and about 1.0 [MPa] in the forceps elevator wire channel. Accordingly, breakage of the endoscope channels due to excessive pressure can be suppressed. At the cleaning and disinfecting steps, originally an object of the air supply is to drain the cleaning water remaining in the endoscope channels. According to the fourth embodiment, since supply of the air to the endoscope channels can be arbitrarily adjusted by adjusting the relief valves 142a to 142c, enough air can be supplied to the endoscope channels.

In the fourth embodiment, the flow rates of air for drainage are measured so that the clogging state of the endoscope channels is detected. This is preferable in that the measurement accuracy of the flow rate sensor 47 can be enhanced, because the flow rate of the air as gas is larger than that of liquid when the supply pressure of the air is equal to that of liquid. The present invention is not, however, limited to this, and the flow rate of the cleaning water or the disinfectant may be measured so that the clogging state is detected. In this case, it is preferable that a pump (not shown) which can supply a fluid with pressure equal to or higher than the resistance pressure of the endoscope channels is provided instead of the pump 45 of the air/water sending system 140. Accordingly, since an enough flow rate of the fluid can be supplied to the endoscope channels, the cleaning effect, the disinfecting effect, the drainage effect, and the like can be improved.

In the fourth embodiment, the compressor 144 which can supply the air with pressure equal to or higher than the resistance pressure of the endoscope channels is used, but the present invention is not limited to this, and a plurality of pumps may be provided so that this function is secured (not shown). The compressor 51 in the first embodiment is used, and a diaphragm unit (not shown) which narrows down the channels for the fluid is provided into the air/water sending system 140, so that the supply pressure of the air may be heightened.

In the fourth embodiment, it is not limited how to read the set values of the relief valves 142a to 142c into the CPU 96 of the control unit. The reading mode includes, for example, (1) when the user directly input the same set values as the set values set for the relief valves 142a to 142c into the control unit, (2) when the control unit is connected with the relief valves 142a to 142c so as to automatically read the set values, and (3) when the same set values as the set values set for the relief valves 142a to 142c are recorded in the nonvolatile memory 19 of the endoscope 2 so as to be read. In the modes (2) and (3), since it is not necessary to input set values for different types of the endoscopes 2, the circulation state of air and fluid supplied to the various endoscope channels can be detected with a simple constitution.

A similar modified example to that of the endoscope cleaning and disinfecting device 1 according to the first embodiment may be applied to the fourth embodiment. For example, the flow rate sensor 47 may be replaced by a pressure sensor, so that the flow rate of not air but of the cleaning water or the disinfectant may be measured. Further, the installation positions of the flow rate sensor 47, the relief valves 142a to 142c, the relief piping 143, and the other components can be changed suitably within a range of obviousness of the person skilled in the art as long as the above working effects can be obtained. These points are applied also to embodiments and modified examples, mentioned later.

Recently, in the field of medical equipment, it is required that not only the clogging of the endoscope channels but also a degree of the clogging should be detected accurately. In order to detect a degree of the clogging, however, it is necessary to detect the clogging states of all the endoscope channels accurately. In the fourth embodiment, since the clogging state in the endoscope channels with high resistance can be detected accurately in the above constitution, the constitution can cope with such a requirement. The constitution, in which a degree of the clogging is detected and this is notified to the user, includes, for example, a constitution in which the comparison calculation steps (S8 and S10) in the first embodiment is further segmented, the comparison calculation steps are executed on a ratio of the set values to the measured values of the flow rates at a plurality of stages, and the calculated results are notified to the user by displaying the results gradually. Specifically, the comparison calculation steps are executed in every 5[%] from 80[%] to 120[%], and the user is notified according to the following table. A red/ blue/yellow lamp (not shown in figures) is provided to the operation panel 85.

| Results of the comparison calculation | Contents to be notified to the user |
|---|---|
| step | and continuation/half of the cleaning |
| | and disinfecting steps |
| Equal to ore less than 80[%] or equal | Buzzer ON |
| to ore more than 120[%] | Display message code |
| | Halt the step |
| 80[%] to 85[%], or 115[%] to | Display red lamp |
| 120[%] | Continue the step |
| 85[%] to 90[%], or 110[%] to | Display yellow lamp |
| 115[%] | Continue the step |
| 90[%] to 110[%] | Display blue lamp |
| | Continue the step |

Accordingly, since not only presence of the clogging of the endoscope channels but also a degree of the clogging can be notified to the user, this constitution can suitably cope with user's demand. Further, since not only the clogging state but also the cleaning and disinfecting state in the endoscope can be notified, the reliability of the endoscope cleaning and disinfecting device can be enhanced. In this constitution, when, for example, the contents to be notified to the user are other than display of the blue lamp, the cleaning and rinsing steps (S4 and S5) are again executed, and the comparison calculation steps and the notifying step may be executed.

### (Fifth Embodiment)

In order to solve the problem similar to the fourth embodiment, the following constitution may be adopted. Fig. 15 is a constitutional diagram of the endoscope cleaning and disinfecting device according to an embodiment of the present invention. In Fig. 15, the same components as those in the first and the fourth embodiments are designated by the same reference signs, and explanations thereof are omitted. The endoscope cleaning and disinfecting device 150 is constituted so that a relief valve 152 in which its opening/closing can be electrically controlled is added to the endoscope cleaning and disinfecting device 1 of the first embodiment. The relief valve 152 is provided on a lower stream side of the flow rate sensor 47 on the piping section 49 of the air/water sending system 151. The relief valve 152 has a solenoid type on-off valve, and is connected with the control unit (not shown in figures) so that the opening/closing of the valve is electrically controlled.

In the fifth embodiment, the relief valve 152 adjusts the pressure of the air supplied from the compressor 144 through the opening/closing of the valve. Air which remains due to the opening and closing of the valve is discharged via the relief piping 153. The air pressure is set individually according to properties of the endoscope channels. The set values are recorded as the channel information in the nonvolatile memory 19 of the endoscope 2, and are read by the CPU 96 of the control unit prior to the cleaning and disinfecting steps (see S1). The air whose pressure is adjusted to a predetermined pressure by the relief valve 152 is sequentially supplied to the endoscope channels via the electromagnetic valves 48a to 48c which sequentially open and close. The clogging state of the endoscope channels is detected (see S6 to S10). According to the fifth embodiment, since the pressure of the air to be supplied can be adjusted in each endoscope channel, a sufficient flow rate of the air can be supplied to even the endoscope channels whose resistance is comparatively high. Accordingly, the measurement accuracy of the flow rate sensor 47 can be enhanced. In the fifth embodiment, the air pressure is set to values equal to or less than the resistance pressure of the endoscope channels. Accordingly, breakage of the endoscope channels due to excessive pressure can be suppressed effectively.

### (Sixth Embodiment)

In order to solve the similar problem to the fourth embodiment, the following constitution may be adopted. That is to say, in a sixth embodiment, instead of the compressor 51 in the first embodiment, a compressor (not shown) which is connected with the control unit and can control an output variably is provided. The output from the compressor is sequentially switched according to opening and closing of the electromagnetic valves 48a to 48c, and the air is supplied to the endoscope channels at a resistance flow rate or with resistance pressure. The resistance flow rate or the resistance pressure of the endoscope channels are known values set in advance, and are recorded as the flow rate information in the nonvolatile memory 19 of the endoscope 2, so as to be read by the CPU 96 of the control unit at the cleaning and disinfecting steps (S3). The presence of clogging in the endoscope channels is determined based on the information about the flow rates read by the CPU 96 (S6 to S11).

According to the sixth embodiment, since enough air can be supplied even to the endoscope channel where the resistance is higher than that of the other channels and a fluid hardly flows, the flow rate can be suitably measured. The compressor is electrically controlled, so that the pressure of the air to be supplied can be controlled so as to be equal to or less than the resistance pressure of the endoscope channels. For this reason, breakage of the endoscope channels due to excessive pressure can be suppressed effectively.

In the sixth embodiment, the resistance flow rate or the resistance pressure is recorded in advance as the information about the flow rate in the nonvolatile memory 19, and the output from the compressor is switched sequentially based on the information about the flow rate. The sequential control is preferable in that the constitution of the control system can be simplified. The present invention is not limited to this, however, the output from the compressor may be controlled in a feedback manner based on the measured results by the flow rate sensor 47 so that the air supply flow rate or supply pressure may be optimized. Accordingly, the flow rate of the air passing through the endoscope channels can be adjusted to a flow rate measurable by the flow rate sensor 47, and breakage of the endoscope channels due to excessive supply of the air can be suppressed effectively.

In the sixth embodiment, a pressure sensor is provided instead of the flow rate sensor 47 or both of them are provided so as to make the above feedback control. Fig. 16 is a constitutional diagram of one example of a modified example. In Fig. 16, like components as those in the first embodiment are designated by like reference signs, and explanations thereof are omitted. The endoscope cleaning and disinfecting device 160 has a pressure sensor 162 on a lower stream side of the flow rate sensor 47 on the piping section 49 of the air/water sending system 161. The compressor 163 can variably change an output, and is connected with the control unit (not shown). The output from the compressor 163 is controlled via the control unit based on the measured results of the flow rate sensor 47 and the pressure sensor 162. Accordingly, the flow rate or the pressure of the air to be supplied to the endoscope channels can be optimized. The constitution using the flow rate sensor 47 is suitable mainly for the detection of the clogging state of the endoscope channels. This is because the clogging state can be detected directly by the flow rate of the fluid. The constitution using the pressure sensor 162 is suitable for preventing the breakage of the endoscope channels. This is because the breakage is mostly caused by excessive pressure. According to the constitution, therefore, since the flow rate sensor 47 and the pressure sensor 162 are provided, the constitution is advantageous to both of the above cases.

### (Seventh Embodiment)

In order to solve the problem similar to that in the fourth embodiment, the following constitution may be adopted. Fig. 17 is a constitutional diagram of the endoscope cleaning and disinfecting device according to the seventh embodiment of the present invention. In Fig. 17, the same components as those in the first embodiment are designated by the same reference signs, and explanations thereof are omitted. In the endoscope cleaning and disinfecting device 170, the flow rate sensor 47 of the endoscope cleaning and disinfecting device 1 in the first embodiment is omitted, and the flow rate sensors 174 and 173, and the pressure sensor 174 are provided on the branched piping 44a to 44c. The flow rate sensor 172 is provided on the channel led to the suction channel 57, and the flow rate sensor 173 is provided on the channel led to the air/water sending channel 56. They are connected with the control unit and function similarly to the flow rate sensor 47 in the first embodiment, so as to measure the flow rates of the passing air. Accordingly, the clogging states of the suction channel 57 and the air/water sending channel 56 are detected.

On the other hand, the pressure sensor 174 is provided on the channel led tot the forceps elevator wire channel and is connected with the control unit so as to measure the presser of the passing air. The clogging state of the forceps elevator wire channel is detected according to the procedure similar to the flow rate measurement in the first embodiment (see Fig. 9). Specifically, for example, a set value Z of the air pressure recorded in the nonvolatile memory 19 of the endoscope 2 is resistance pressure of the forceps elevator wire channel, and a known value is used. The set value is read by the CPU 96 of the control unit (see S3) prior to the cleaning step (S4). A measured value z of the forceps elevator wire channel becomes a measured value of the pressure sensor 174 (see S6). A ratio C of the set value Z to the set value z is calculated (see S7), and the comparison calculation steps and the notifying step are executed based on the calculated result (see S8 to S11). When the ratio C is smaller than 80[%] (see S8 and S9), the fluid supply pressure is low, and the air is in danger of being leaked in either channel. Further, when the ratio C is larger than 120[%] (see S10 and S11), the fluid supply pressure is high and the forceps elevator wire channel is in danger of being clogged. At this step, the pressure sensor 174 can detect the clogging state of the forceps elevator wire channel.

In the forceps elevator wire channel, since the resistance of the channel is high and a fluid hardly flows, it is difficult to measure the flow rate accurately. In the seventh embodiment, since the pressure sensor 174 measures the pressure of the supply fluid and detect the clogging state of the forceps elevator wire channel, even when the air flow rate is small, a change in the air passing state can be detected accurately. Accordingly, the detection using the pressure sensor 174 can be more accurate than the detecting using the flow rate sensor 47.

In the seventh embodiment, the flow rate sensors 172 and 173 may be constituted as a single flow rate sensor (not shown in figures). Accordingly, a number of the components can be reduced. From the similar viewpoint, when a plurality of endoscope channels are present, one flow rate sensor and one pressure sensor are suffice by devising the constitution of the branched piping 44 (see Fig. 18). Accordingly, a number of sensors to be installed can be reduced.

### (Eighth Embodiment)

In order to solve the problem similar to that in the fourth embodiment, the following constitution may be adopted. Fig. 19 is a constitutional diagram of the endoscope cleaning and disinfecting device according to an eighth embodiment. In Fig. 19, the same components as those in the first embodiment are designated by the same reference signs, and explanations thereof are omitted. The endoscope cleaning and disinfecting device 180 has the air/water sending system 181 including the flow rate sensor 47, and the air/water sending system 182 including the pressure sensor 183, and they are used according to types of the endoscope channels. The air/water sending system 181 including the flow rate sensor 47 is connected with the suction channel 57 and the air/water sending channel 56. The endoscope channels has such a characteristic that the resistance of the channels is comparatively low and a fluid easily flows. The constitution and the function of the air/water sending system 181 are approximately same as those of the air/water sending system in the first embodiment. The flow rate sensor 47 sequentially measures the flow rates of the air in the suction channel 57 and in the air/water sending channel 56, so as to detect their clogging states.

On the other hand, the air/water sending system 182 including the pressure sensor 183 is newly added to the endoscope cleaning and disinfecting device 1 of the first embodiment. The air/water sending system 182 includes the pressure sensor 183, the compressor 184, and the pump 185. The pressure sensor 183 is provided on the piping section 186, and is connected with the control unit (not shown). A front end of the piping section 186 is connected to the connection port 35 led to the forceps elevator wire channel, and its rear end is branched into two. One of the branched rear ends is opened, and the other end is connected to the circulation liquid suction port 30 of the cleaning tab 29. The compressor 184 as well as the check valve 187 is provided to the opened end of the piping section 186. The pump 185 as well as the check valve 188 is provided on the piping section 186 on the side of the circulation liquid suction port 30. The air/water sending system 182 has the approximately same function as that of the other air/water sending system 181, but has such a characteristic that the pressure sensor 183 measures the air pressure so as to detect the clogging state of the forceps elevator wire channel. Accordingly, the clogging state of the forceps elevator wire channel with high resistance of channel can be measured accurately regardless of the flow rate of the air.

In the eighth embodiment, the air/water sending systems 181 and 182 are individually provided according to a difference in the resistance of the endoscope channels, and the sensors 47 and 183 suitably detect clogging states. Accordingly, the clogging states can be detected accurately. According to the eighth embodiment, the specifications of the compressors 51 and 184 can be specialized according to measuring ranges of the sensors 47 and 183. For example, the pressure of the compressor 51 may be low as long as the flow rate of a fluid to be supplied is large. This is because when the flow rate of the fluid can have a certain value in the endoscope channels having comparatively low resistance, the flow rate sensor 47 can detect the clogging state accurately. On the other hand, the flow rate of the compressor 184 may be small as long as the pressure of a fluid to be supplied is high. This is because when the flow rate of the fluid has a certain value in the channels of the endoscope having comparatively high resistance, the pressure sensor 183 can suitably detect the clogging states regardless of a degree of the flow rate. If a single air/water sending system detects the clogging states of all the endoscope channels, however, a compressor which can supply a large flow rate of a fluid with high pressure is required. Such a compressor is generally expensive. According to the eighth embodiment, therefore, the specifications of the compressors 51 and 184 can be specialized according to the characteristics of the endoscope channels. For this reason, the inexpensive compressors 51 and 184 can compose the air/water sending system. Further, since the compressors 51 and 184 which are suited for objects to be measured and the measuring ranges of the sensors 47 and 183 can be selected, accurately measured values can be obtained.

In the eighth embodiment, since the endoscope channels have different resistance, if a single pump supplies a fluid, similarly to the above case of the compressor, a high-performance pump which can supply a large flow rate of a fluid with high pressure is required. In the eighth embodiment, the suitable pumps 45 and 185 are individually provided according to a difference in resistance of the endoscope channels. Accordingly, since pumps which have a necessary specification can be suitably selected, the entire cost of the endoscope cleaning and disinfecting device 18 can be reduced. Since suitable pumps can be selected according to the characteristics of the endoscope channels, the cleaning property and the disinfecting property can be improved.

In the eighth embodiment, the flow rate sensor 47 is used for the endoscope channel where the resistance is comparatively small and a fluid easily flows. This is because when the clogging states of the endoscope channels are detected, the measurement of the flow rates which is a direct and certain method is preferably used. The detection of the clogging states is not limited to this, however, the pressure sensor may be provided instead of the flow rate sensor 47, so as to detect the clogging states of the endoscope channels.

In the eighth embodiment, the similar modified example to the modified examples in the first to the seventh embodiments may be adopted. For example, both the flow rate sensor and the pressure sensor may be provided to the air/water sending systems 181 and 182. Accordingly, either sensor which performs a measurement easily can detect the clogging states of the endoscope channels. For example, the supply flow rate and the supply pressure of not air but cleaning water and disinfectant may be measured by the sensors 47 and 183. In this case, the pumps 45 and 185 supply a fluid. Accordingly, since a pump which is appropriate to the characteristics of the endoscope channels can be selected, the cleaning property and the disinfecting property can be improved.

In the eighth embodiment, the air/water sending systems 181 and 182 may be sequentially operated by using a single control unit, or may be simultaneously operated by individual control units. The former method is preferable in that a new control unit does not have to be provided, and the latter method is preferable in that measuring duration can be shortened. In the eighth embodiment, the air/water sending systems 181 and 182 have the pumps 45 and 185, respectively, but they may share single pump (see Fig. 20). For example, when the endoscope channels are cleaned and disinfected, the method using single pump is adapted to the case where the use of the pumps having different specifications is less useful.

In the eighth embodiment, when a plurality of endoscope channels with high resistance are present, the air/water sending system 182 may be constituted as follows. Fig. 21 is a constitutional diagram a modified example. As shown in Fig. 21, a plurality of electromagnetic valves 48d to 48f are provided also on the air/water sending system 182, and they may be sequentially switched so that the clogging states of the endoscope channels are detected. This can cope with the case where a plurality of the endoscope channels having different characteristics are present.

### (Ninth Embodiment)

In the first to the eighth embodiments, the clogging states of the endoscope channels are detected after the rinsing step (S5), but the order of the step is not limited to this, and prior to the cleaning step (S4), the clogging states may be detected. The step of cleaning and disinfecting a clogged endoscope channel can be omitted. When clogging occurs, breakage of the endoscope channel due to supply of the cleaning water can be prevented. An endoscope where clogging is found is subject to another maintenance step. Further, when clogging is not detected as a result of the detection, outputs form the pumps are raised to a durability limit of the endoscope channels, so that the endoscope channels is cleaned. Accordingly, since the cleaning and disinfecting at the durability limit can be executed, the cleaning property and the disinfecting property of the endoscope channels can be improved.

### INDUSTRIAL APPLICABILITY

The endoscope cleaning and disinfecting device and the endoscope cleaning and disinfecting method adjust the pressure and the flow rates of a fluid to be supplied individually according to the characteristics of the endoscope channels. For this reason, the device and the method are effective in that the measurement accuracy of the pressure or the flow rates of the fluid can be enhanced, and the clogging states of the endoscope channels can be detected accurately.

## Claims

1. An endoscope cleaning and disinfecting device (1) that cleans and disinfects an endoscope (2) including plural types of channels having different fluid passing characteristics using a fluid, comprising:
a fluid supply unit (42 - 45, 49, 51) that supplies the fluid for cleaning and disinfecting the endoscope to the endoscope channels;
a measuring unit (47, 162) that measures pressure or flow rates of the fluid flowing through the endoscope channels; and
a detecting unit (85, 96) that performs a comparison calculation based on measured values obtained by the measuring unit (47, 162) and predetermined set values for each of the endoscope channels according to the fluid passing characteristics of the channels so as to detect clogging states of the endoscope channels, wherein the set values are set so that the measuring unit can measure the pressure or the flow rates of the fluid;
**characterized in that** the fluid supply unit includes an adjustment unit (48, 96, 99, 102, 142, 152) that adjusts the pressure or the flow rate of the fluid to the predetermined set values for each of the endoscope channels according to the fluid passing characteristics of the endoscope channels.

2. The endoscope cleaning and disinfecting device (1) according to claim 1, wherein when resistance of one endoscope channel is higher than that of another endoscope channel, the adjustment unit (48, 96, 99, 102, 142, 152) adjusts the pressure of the fluid to be supplied to the endoscope channel having higher resistance, so that the pressure is adjusted higher than that of another endoscope channel.

3. The endoscope cleaning and disinfecting device (1) according to claim 1, wherein when resistance of one endoscope charnel is higher than that of another endoscope channel, the measuring unit for the endoscope channel having higher pressure is a pressure measuring unit (162) that measures the pressure of the fluid.

4. The endoscope cleaning and disinfecting device (1) according to claim 1, wherein the predetermined set values are set so as to be equal to or less than a durability limit of the endoscope channels.

5. The endoscope cleaning and disinfecting device (1) according to claim 1, wherein the fluid supply adjustment unit (48, 142, 152) is arranged on a fluid channel (49, 44a - 44c) and adjusts the pressure or the flow rate of the passing fluid individually to the predetermined set value for each of the endoscope channels.

6. The endoscope cleaning and disinfecting device (1) according to claim 1, wherein the adjustment unit (48, 96, 99, 102, 142, 152) is a variable supplying unit adapted to variably control the pressure or the flow rate of the fluid to be supplied according to the fluid passing characteristics of the endoscope channels.

7. The endoscope cleaning and disinfecting device (1) according to any one of claims 1 to 6, wherein the detecting unit (85, 96) includes a notifying unit (92, 94) that determines whether the measured values are within a predetermined range of the set values and notifies determined results gradually.

8. The endoscope cleaning and disinfecting device (1) according to any one of claims 1 to 6, wherein the detecting unit (85, 96) has a reading unit that reads the set values and the detecting unit performs the comparison calculation using the read set values.

9. The endoscope cleaning and disinfecting device (1) according to any one of claims 1 to 6, wherein
the set values are preset in the endoscope to be cleaned and disinfected, and
the detecting unit has a reading unit that reads the set values and the detecting unit performs the comparison calculation using the read set values.

10. The endoscope cleaning and disinfecting device (1) according to any one of claims 1 to 6, wherein the fluid supply unit (42 - 45, 49, 51) has an air sending source (49, 51) that sends air to the endoscope channels, a cleaning tube that is connected with the endoscope channels, a changeover unit that switches the endoscope channels to which the air is sent, and a unit that connects the air sending channel and the water sending channel in the endoscope.

11. The endoscope cleaning and disinfecting device (1) according to any one of claims 1 to 6, wherein the detecting unit (85, 96) includes a notifying unit (92, 94) that, when the measured values are within a predetermined range of the set values, notifies the state.

12. The endoscope cleaning and disinfecting device according to claim 7, wherein the contents to be notified includes states such as the flow rate decreases in a certain place of the endoscope channels, a cleaning tube is disconnected, the cleaning tube is forgotten to be attached, piping in the device is disconnected, a leakage occurs in the piping in the device, or the air sending source in the device is abnormally operated.

13. The endoscope cleaning and disinfecting device (1) according to any one of claims 1 to 6, wherein the fluid to be supplied to the endoscope channels is a liquid for cleaning and disinfecting, the fluid supply unit has a water sending piping (44, 53, 54, 55) that leads the liquid to the endoscope channels, and a control unit that controls the liquid flowing through the endoscope channels based on the comparison calculation results is further provided.

14. The endoscope cleaning and disinfecting device (1) according to claim 13, further including a notifying unit (92, 94) that notifies a control state of the control unit based on the compared result of the comparing unit.

15. The endoscope cleaning and disinfecting device (1) according to claim 13, comprising a first connector that can be connected detachably with an opening at one end of the first endoscope channel, a second connector that can be connected detachably with an opening at the other end of the first endoscope channel, a third connector that can be connected detachably with an opening at one end of the second endoscope channel, and a connecting unit that connects the second connector and the third connector, these units being provided on the water sending piping.

16. The endoscope cleaning and disinfecting device (1) according to claim 13, comprising a first liquid sending piping connected with an end of the first endoscope channel and an end of the water sending piping, a second liquid sending piping connected with an end of the second endoscope channel and an end of the water sending piping, a plurality of electromagnetic valves provided on the first liquid sending piping and the second liquid sending piping respectively, and a control unit that selectively switches the electromagnetic valves.

17. A method of cleaning and disinfecting an endoscope including plural types of channels having different fluid passing characteristics using a fluid, comprising:
a fluid supplying step of supplying the fluid for cleaning and disinfecting the endoscope to the endoscope channels;
a fluid supply adjustment step of adjusting pressure or flow rate of the fluid to a predetermined set value for each of the endoscope channels according to the fluid passing characteristics of the endoscope channels;
a measuring step of measuring the pressure or flow rate of the fluid flowing through the endoscope channels; and
a detecting step of performing a comparison calculation based on measured values obtained by the measuring step and predetermined set values for each of the endoscope channels according to the fluid passing characteristics of the channels so as to detect clogging states of the endoscope channels, wherein the predetermined set values are set so that the measuring unit can measure the pressure or the flow rates of the fluid.

18. The method according to claim 17, wherein the detecting step is executed prior to cleaning and disinfecting steps for the endoscope channels.

19. The method according to claim 18, further comprising a durability limit cleaning step of, when clogging of the endoscope channels is within a predetermined range as a result of detecting the clogging states, heightening the pressure or the flow rates of the fluid to be supplied to the endoscope channels up to a durability limit of the endoscope channels and of cleaning the endoscope channels.

20. The method according to any one of claims 17 to 19, wherein the detecting step includes a reading step of reading the set values, and the comparison calculation is executed by using the read set values at the detecting step.

21. The method according to any one of claims 17 to 20, wherein the detecting step includes a notifying step of, when the measured values are within a predetermined range of the set values, notifying the state.

22. The endoscope cleaning and disinfecting device according to claim 1, wherein:
the plural types of channels include a first endoscope channel group whose resistance is lower than that of the other channel groups, and a second endoscope channel group whose resistance is higher than that of the other channel groups;
the fluid supply unit includes a first fluid supply unit that supplies fluid to the first endoscope channel group, and a second fluid supply unit that supplies fluid to the second endoscope channel group;
the measuring unit includes a first measuring unit (47) that measures flow rates of the fluid flowing through the first endoscope channels and a second measuring unit (183) that measures pressure of the fluid flowing through the second endoscope channel group; and
the first fluid supply unit and the first measuring unit form a first measuring system (181); and
the second fluid supply unit and the second measuring unit form a second measuring system (182).

## Patentansprüche

1. Eine Vorrichtung (1) zum Waschen und Desinfizieren eines Endoskops, welche ein Endoskop (2), das eine Mehrzahl von Kanaltypen mit unterschiedlichen Fluiddurchlasseigenschaften hat, unter Verwendung eines Fluids wäscht und desinfiziert, aufweisend:
eine Fluidzufuhreinheit (42-45, 49, 51), die das Fluid zum Waschen und Desinfizieren des Endoskops den Endoskopkanälen zuführt;
eine Messeinheit (47, 162), welche den Druck oder Flussraten des Fluids misst, das durch die Endoskopkanäle fließt; und
eine Erkennungseinheit (85, 96), die eine Vergleichsberechnung, basierend auf von der Messeinheit (47, 162) erhaltenen, gemessenen Werten und vorbestimmten Setzwerten für jeden der Endoskopkanäle gemäß den Fluiddurchlasseigenschaften der Kanäle durchführt, um Verstopfungszustände der Endoskopkanäle zu erkennen, wobei die Setzwerte so gesetzt sind, dass die Messeinheit den Druck oder die Flussraten des Fluids messen kann;
**dadurch gekennzeichnet, dass** die Fluidzufuhreinheit eine Einstelleinheit (48, 96, 99, 102, 142, 152) enthält, welche den Druck oder die Flussrate des Fluids auf die vorbestimmten Setzwerte für jeden der Endoskopkanäle gemäß den Fluiddurchlasseigenschaften der Endoskopkanäle einstellt.

2. Die Vorrichtung (1) zum Waschen und Desinfizieren eines Endoskops nach Anspruch 1, bei der, wenn ein Widerstand in einem der Endoskopkanäle höher als der eines anderen Endoskopkanals ist, die Einstelleinheit (48, 96, 99, 102, 142, 152) den Druck des Fluids, das dem Endoskopkanal zuzuführen ist, der höheren Widerstand hat, einstellt, so dass der Druck auf höher als derjenige des anderen Endoskopkanals eingestellt wird.

3. Die Vorrichtung (1) zum Waschen und Desinfizieren eines Endoskops nach Anspruch 1, wobei, wenn der Widerstand eines Endoskopkanals höher als derjenige eines anderen Endoskopkanals ist, die Messeinheit für den Endoskopkanal, der höheren Druck hat, eine Druckmesseinheit (162) ist, die den Druck des Fluids misst.

4. Die Vorrichtung (1) zum Waschen und Desinfizieren eines Endoskops nach Anspruch 1, bei der die vorbestimmten Setzwerte so gesetzt werden, dass sie gleich oder kleiner als eine Haltbarkeitsgrenze der Endoskopkanäle sind.

5. Die Vorrichtung (1) zum Waschen und Desinfizieren eines Endoskops nach Anspruch 1, bei der die Fluidzufuhreinstelleinheit (48, 142, 152) an einem Fluidkanal (49, 44a - 44c) angeordnet ist und den Druck oder die Flussrate des durchlaufenden Fluids individuell auf den vorbestimmten Setzwert für jeden der Endoskopkanäle einstellt.

6. Die Vorrichtung (1) zum Waschen und Desinfizieren eines Endoskops nach Anspruch 1, wobei die Einstelleinheit (48, 96, 99, 102, 142, 152) eine variable Zufuhreinheit ist, welche variabel den Druck oder die Flussrate des zuzuführenden Fluids gemäß den Fluiddurchlasseigenschaften der Endoskopkanäle zu steuern vermag.

7. Die Vorrichtung (1) zum Waschen und Desinfizieren eines Endoskops nach einem der Ansprüche 1 bis 6, wobei die Erkennungseinheit (85, 96) eine Meldeeinheit (92, 94) enthält, die bestimmt, ob die gemessenen Werte innerhalb eines bestimmten Bereichs der Setzwerte sind und die bestimmten Ergebnisse nach und nach meldet.

8. Die Vorrichtung (1) zum Waschen und Desinfizieren eines Endoskops nach einem der Ansprüche 1 bis 6, bei der die Erkennungseinheit (85, 96) eine Leseeinheit hat, welche die Setzwerte liest, wobei die Erkennungseinheit die Vergleichsberechnung unter Verwendung der gelesenen Setzwerte durchführt.

9. Die Vorrichtung (1) zum Waschen und Desinfizieren eines Endoskops nach einem der Ansprüche 1 bis 6, bei der die Setzwerte in dem zu waschenden und desinfizierenden Endoskops festgesetzt sind und die Erkennungseinheit eine Leseeinheit hat, welche die Setzwerte liest und wobei die Erkennungseinheit die Vergleichsberechnung unter Verwendung der gelesenen Setzwerte durchführt.

10. Die Vorrichtung (1) zum Waschen und Desinfizieren eines Endoskops nach einem der Ansprüche 1 bis 6, bei der die Fluidzufuhreinheit (42-45, 49, 51) eine Luftsendequelle (49, 51) hat, welche Luft zu den Endoskopkanälen sendet, sowie eine Reinigungsröhre, die mit den Endoskopkanälen verbunden ist, eine Umschalteinheit, die die Endoskopkanäle schaltet, zu welchen Luft gesendet wird und eine Einheit, die den Luftsendekanal und den Wassersendekanal in dem Endoskop verwendet.

11. Die Vorrichtung (1) zum Waschen und Desinfizieren eines Endoskops nach einem der Ansprüche 1 bis 6, bei der die Erkennungseinheit (85, 96) eine Meldeeinheit (92, 94) enthält, welche, wenn die gemessenen Werte innerhalb eines vorbestimmten Bereichs der Setzwerte sind, den Zustand meldet.

12. Die Vorrichtung (1) zum Waschen und Desinfizieren eines Endoskops nach Anspruch 7, bei der der zu meldende Inhalt Zustände enthält wie: die Flussrate nimmt an einer bestimmten Stelle der Endoskopkanäle ab; eine Reinigungsröhre ist getrennt; die Reinigungsröhre wurde vergessen anzubringen; eine Leitungsverbindung in der Vorrichtung ist unterbrochen; ein Leck tritt in der Leitungsverbindung in der Vorrichtung auf; oder die Luftsendequelle in der Vorrichtung wird anormal betrieben.

13. Die Vorrichtung (1) zum Waschen und Desinfizieren eines Endoskops nach einem der Ansprüche 1 bis 6, wobei das den Endoskopkanälen zuzuführende Fluid eine Flüssigkeit zum Waschen und Desinfizieren ist, die Fluidzufuhreinheit eine Wassersendeleitungsverbindung (44, 53, 54, 55), die die Flüssigkeit zu den Endoskopkanälen leitet, aufweist und wobei weiterhin eine Steuereinheit vorgesehen ist, die das Fließen der Flüssigkeit durch die Endoskopkanäle basierend auf den Vergleichsberechnungsergebnissen steuert.

14. Die Vorrichtung (1) zum Waschen und Desinfizieren eines Endoskops nach Anspruch 13, weiterhin mit einer Meldeeinheit (92, 94), die einen Steuerzustand der Steuereinheit basierend auf dem Vergleichsergebnis der Vergleichseinheit meldet.

15. Die Vorrichtung (1) zum Waschen und Desinfizieren eines Endoskops nach Anspruch 13, aufweisend einen ersten Verbinder, der entfernbar mit einer Öffnung an einem Ende des ersten Endoskopkanals verbindbar ist, einen zweiten Verbinder, der entfernbar mit einer Öffnung am anderen Ende des ersten Endoskopkanals verbindbar ist, einem dritten Verbinder, der entfernbar mit einer Öffnung an einem Ende des zweiten Endoskopkanals verbindbar ist und eine Verbindungseinheit, die den zweiten Verbinder und den dritten Verbinder verbindet, wobei diese Einheiten in der Wassersendeleitungsverbindung angeordnet sind.

16. Die Vorrichtung (1) zum Waschen und Desinfizieren eines Endoskops nach Anspruch 13, aufweisend eine erste Flüssigkeitssendeleitungsverbindung in Verbindung mit einem Ende des ersten Endoskopkanals und einem Ende der Wassersendeleitungsverbindung, eine zweite Flüssigkeitssendeleitungsverbindung in Verbindung mit einem Ende des zweiten Endoskopkanals und einem Ende der Wassersendeleitungsverbindung, eine Mehrzahl von elektromagnetischen Ventilen in der ersten Flüssigkeitssendeleitungsverbindung bzw. der zweiten Flüssigkeitssendeleitungsverbindung, und eine Steuereinheit, welche selektiv die elektromagnetischen Ventile schaltet.

17. Ein Verfahren zum Waschen und Desinfizieren eines Endoskops, das eine Mehrzahl von Kanaltypen mit unterschiedlichen Fluiddurchlasseigenschaften hat, unter Verwendung eines Fluids, aufweisend:
einen Fluidzufuhrschritt des Zuführens des Fluids zum Waschen und Desinfizieren des Endoskops zu den Endoskopkanälen;
einen Fluidzufuhreinstellschritt des Einstellens von Druck oder Flussrate des Fluids auf einen vorbestimmten Setzwert für jeden der Endoskopkanäle gemäß den Fluiddurchlasseigenschaften der Endoskopkanäle;
einen Messschritt des Messens des Drucks oder der Flussrate des durch die Endoskopkanäle fließenden Fluids; und
einen Erkennungsschritt des Durchführens einer Vergleichsberechnung basierend auf Messwerten, erhalten durch den Messschritt und vorbestimmten Setzwerten für jeden der Endoskopkanäle gemäß den Fluiddurchlasseigenschaften der Kanäle, um so Verstopfungszustände der Endoskopkanäle zu erkennen, wobei die vorbestimmten Setzwerte so gesetzt sind, dass die Messeinheit den Druck oder die Flussraten des Fluids messen kann.

18. Das Verfahren nach Anspruch 17, wobei der Erkennungsschritt vor den Wasch- und Desinfektionsschritten für die Endoskopkanäle durchgeführt wird.

19. Das Verfahren nach Anspruch 18, weiterhin aufweisend einen Haltbarkeitsgrenzreinigungsschritt, bei dem, wenn eine Verstopfung der Endoskopkanäle als ein Ergebnis der Erkennung der Verstopfungszustände innerhalb eines bestimmten Bereichs ist, der Druck oder die Flussraten des Fluids, das den Endoskopkanälen zugeführt wird, bis zu einer Haltbarkeitsgrenze der Endoskopkanäle erhöht wird und die Endoskopkanäle gewaschen werden.

20. Das Verfahren nach einem der Ansprüche 17 bis 19, bei dem der Erkennungsschritt einen Leseschritt des Lesens der Setzwerte enthält und die Vergleichsberechnung durch Verwendung der im Erkennungsschritt gelesenen Setzwerte durchgeführt wird.

21. Das Verfahren nach einem der Ansprüche 17 bis 20, bei dem der Erkennungsschritt einen Meldeschritt aufweist, bei dem, wenn die Messwerte innerhalb eines bestimmten Bereichs von Setzwerten sind, der Zustand gemeldet wird.

22. Die Vorrichtung zum Waschen und Desinfizieren eines Endoskops nach Anspruch 1, bei der:
die Mehrzahl von Kanaltypen eine erste Endoskopkanalgruppe, deren Widerstand niedriger als der der anderen Kanalgruppen ist und eine zweite Endoskopkanalgruppe umfasst, deren Widerstand höher als derjenige der anderen Kanalgruppen ist;
die Fluidzufuhreinheit eine erste Fluidzufuhreinheit ist, die Fluid der ersten Endoskopkanalgruppe zuführt und eine zweite Fluidzufuhreinheit aufweist, die Fluid der zweiten Endoskopkanalgruppe zuführt;
die Messeinheit eine erste Messeinheit (47), die Flussraten des Fluids misst, welches durch die ersten Endoskopkanäle fließt und eine zweite Messeinheit (183) aufweist, die den Druck des Fluids misst, das durch die zweite Endoskopkanalgruppe fließt; und
die erste Fluidzufuhreinheit und die erste Messeinheit ein erstes Messsystem (181) bilden und die zweite Fluidzufuhreinheit und die zweite Messeinheit ein zweites Messsystem (182) bilden.

## Revendications

1. Dispositif (1) de nettoyage et de désinfection d'endoscope qui nettoie et désinfecte un endoscope (2) incluant des types pluraux de canaux ayant des caractéristiques de passage de fluide différentes en utilisant un fluide, comprenant :
une unité d'amenée de fluide (42-45, 49, 51) qui amène le fluide pour nettoyer et désinfecter l'endoscope jusqu'aux canaux d'endoscope ;
une unité de mesure (47, 162) qui mesure la pression ou les débits du fluide s'écoulant à travers les canaux d'endoscope ; et
une unité de détection (85, 96) qui effectue un calcul de comparaison sur la base des valeurs mesurées obtenues par l'unité de mesure (47, 162) et de valeurs fixées prédéterminées pour chacun des canaux d'endoscope en fonction des caractéristiques de passage de fluide des canaux de manière à détecter des états de colmatage des canaux d'endoscope, dans lequel les valeurs fixées sont fixées de manière à ce que l'unité de mesure puisse mesurer la pression ou les débits du fluide ;
**caractérisé en ce que** l'unité d'amenée de fluide inclut une unité d'ajustement (48, 96, 99, 102, 142, 152) qui ajuste la pression ou le débit du fluide aux valeurs fixées prédéterminées pour chacun des canaux d'endoscope en fonction des caractéristiques de passage de fluide des canaux d'endoscope.

2. Dispositif (1) de nettoyage et de désinfection d'endoscope selon la revendication 1, dans lequel lorsqu'une résistance d'un canal d'endoscope est supérieure à celle d'un autre canal d'endoscope, l'unité d'ajustement (48, 96, 99, 102, 142, 152) ajuste la pression du fluide devant être amené au canal d'endoscope ayant une résistance plus élevée, de sorte que la pression est ajustée à une valeur supérieure à celle d'un autre canal d'endoscope.

3. Dispositif (1) de nettoyage et de désinfection d'endoscope selon la revendication 1, dans lequel lorsqu'une résistance d'un canal d'endoscope est supérieure à celle d'un autre canal d'endoscope, l'unité de mesure pour le canal d'endoscope ayant une pression plus élevée est une unité de mesure de pression (162) qui mesure la pression du fluide.

4. Dispositif (1) de nettoyage et de désinfection d'endoscope selon la revendication 1, dans lequel les valeurs fixées prédéterminées sont fixées de manière à être égales ou inférieures à une limite de durabilité des canaux d'endoscope.

5. Dispositif (1) de nettoyage et de désinfection d'endoscope selon la revendication 1, dans lequel l'unité d'ajustement d'amenée de fluide (48, 142, 152) est agencée sur un canal de fluide (49, 44a-44c) et ajuste la pression ou le débit du fluide passant individuellement à la valeur fixée prédéterminée pour chacun des canaux d'endoscope.

6. Dispositif (1) de nettoyage et de désinfection d'endoscope selon la revendication 1, dans lequel l'unité d'ajustement (48, 96, 99, 102, 142, 152) est une unité d'amenée variable adaptée pour commander de manière variable la pression ou le débit du fluide devant être amené en fonction des caractéristiques de passage de fluide des canaux d'endoscope.

7. Dispositif (1) de nettoyage et de désinfection d'endoscope selon l'une quelconque des revendications 1 à 6, dans lequel l'unité de détection (85, 96) inclut une unité de notification (92, 94) qui détermine si les valeurs mesurées sont à l'intérieur d'une plage prédéterminée des valeurs fixées et notifie les résultats déterminés graduellement.

8. Dispositif (1) de nettoyage et de désinfection d'endoscope selon l'une quelconque des revendications 1 à 6, dans lequel l'unité de détection (85, 96) a une unité de lecture qui lit les valeurs fixées, et l'unité de détection effectue le calcul de comparaison en utilisant les valeurs fixées lues.

9. Dispositif (1) de nettoyage et de désinfection d'endoscope selon l'une quelconque des revendications 1 à 6, dans lequel
les valeurs fixées sont pré-fixées dans l'endoscope devant être nettoyé et désinfecté, et
l'unité de détection a une unité de lecture qui lit les valeurs fixées, et l'unité de détection effectue le calcul de comparaison en utilisant les valeurs fixées lues.

10. Dispositif (1) de nettoyage et de désinfection d'endoscope selon l'une quelconque des revendications 1 à 6, dans lequel l'unité d'amenée de fluide (42-45, 49, 51) a une source d'envoi d'air (49, 51) qui envoie de l'air dans les canaux d'endoscope, un tube de nettoyage qui est connecté avec les canaux d'endoscope, une unité d'inversion qui commute les canaux d'endoscope dans lesquels l'air est envoyé, et une unité qui connecte le canal d'envoi d'air et le canal d'envoi d'eau dans l'endoscope.

11. Dispositif (1) de nettoyage et de désinfection d'endoscope selon l'une quelconque des revendications 1 à 6, dans lequel l'unité de détection (85, 96) inclut une unité de notification (92, 94) qui, lorsque les valeurs mesurées sont à l'intérieur d'une plage prédéterminée des valeurs fixées, notifie l'état.

12. Dispositif (1) de nettoyage et de désinfection d'endoscope selon la revendication 7, dans lequel le contenu destiné à être notifié inclut des états tels que le débit diminue dans un certain endroit des canaux d'endoscope, un tube de nettoyage est déconnecté, le tube de nettoyage a été oublié d'être relié, une canalisation dans le dispositif est déconnectée, il y a une fuite dans les canalisations dans le dispositif ou bien la source d'envoi d'air dans le dispositif est utilisée de façon anormale.

13. Dispositif (1) de nettoyage et de désinfection d'endoscope selon l'une quelconque des revendications 1 à 6, dans lequel le fluide destiné à être amené dans les canaux d'endoscope est un liquide pour le nettoyage et la désinfection, l'unité d'amenée de fluide a une canalisation d'envoi d'eau (44, 53, 54, 55) qui conduit le liquide dans les canaux d'endoscope, et une unité de commande qui commande le liquide s'écoulant à travers les canaux d'endoscope sur la base des résultats du calcul de comparaison est en outre prévue.

14. Dispositif (1) de nettoyage et de désinfection d'endoscope selon la revendication 13, incluant en outre une unité de notification (92, 94) qui notifie un état de commande de l'unité de commande sur la base du résultat comparé de l'unité de comparaison.

15. Dispositif (1) de nettoyage et de désinfection d'endoscope selon la revendication 13, comprenant un premier connecteur qui peut être connecté de façon détachable avec une ouverture à une extrémité du premier canal d'endoscope, un deuxième connecteur qui peut être connecté de façon détachable avec une ouverture à l'autre extrémité du premier canal d'endoscope, un troisième connecteur qui peut être connecté de façon détachable avec une ouverture à une extrémité du deuxième canal d'endoscope, et une unité de connexion qui connecte le deuxième connecteur et le troisième connecteur, ces unités étant prévues sur la canalisation d'envoi d'eau.

16. Dispositif (1) de nettoyage et de désinfection d'endoscope selon la revendication 13, comprenant une première canalisation d'envoi de liquide connectée avec une extrémité du premier canal d'endoscope et une extrémité de la canalisation d'envoi d'eau, une deuxième canalisation d'envoi de liquide connectée avec une extrémité du deuxième canal d'endoscope et une extrémité de la canalisation d'envoi d'eau, une pluralité de soupapes électromagnétiques prévues sur la première canalisation d'envoi de liquide et la deuxième canalisation d'envoi de liquide respectivement, et une unité de commande qui commute de façon sélective les soupapes électromagnétiques.

17. Procédé de nettoyage et de désinfection d'un endoscope incluant des types pluraux de canaux ayant des caractéristiques de passage de fluide différentes en utilisant un fluide, comprenant :
une étape d'amenée de fluide qui amène le fluide pour nettoyer et désinfecter l'endoscope jusqu'aux canaux d'endoscope ;
une étape d'ajustement d'amenée de fluide qui ajuste la pression ou le débit du fluide à une valeur fixée prédéterminée pour chacun des canaux d'endoscope en fonction des caractéristiques de passage de fluide des canaux d'endoscope ;
une étape de mesure qui mesure la pression ou le débit du fluide s'écoulant à travers les canaux d'endoscope ; et
une étape de détection qui effectue un calcul de comparaison sur la base de valeurs mesurées obtenues par l'étape de mesure et de valeurs fixées prédéterminées pour chacun des canaux d'endoscope en fonction des caractéristiques de passage de fluide des canaux de manière à détecter des états de colmatage des canaux d'endoscope, dans lequel les valeurs fixées prédéterminées sont fixées de manière à ce que l'unité de mesure puisse mesurer la pression ou les débits du fluide.

18. Procédé selon la revendication 17, dans lequel l'étape de détection est exécutée avant les étapes de nettoyage et de désinfection pour les canaux d'endoscope.

19. Procédé selon la revendication 18, comprenant en outre une étape de nettoyage à limite de durabilité qui, lorsque le colmatage des canaux d'endoscope est à l'intérieur d'une plage prédéterminée en résultat de la détection des états de colmatage, augmente la pression ou les débits du fluide destiné à être amené dans les canaux d'endoscope jusqu'à une limite de durabilité des canaux d'endoscope et de nettoyage des canaux d'endoscope.

20. Procédé selon l'une quelconque des revendications 17 à 19, dans lequel l'étape de détection inclut une étape de lecture qui lit les valeurs fixées et le calcul de comparaison est exécuté en utilisant les valeurs fixées lues lors de l'étape de détection.

21. Procédé selon l'une quelconque des revendications 17 à 20, dans lequel l'étape de détection inclut une étape de notification qui, lorsque les valeurs mesurées sont à l'intérieur d'une plage prédéterminée des valeurs fixées, notifie l'état.

22. Dispositif de nettoyage et de désinfection d'endoscope selon la revendication 1, dans lequel :
les types pluraux de canaux incluent un premier groupe de canaux d'endoscope dont la résistance est inférieure à celle des autres groupes de canaux, et un deuxième groupe de canaux d'endoscope dont la résistance est supérieure à celle des autres groupes de canaux ;
l'unité d'amenée de fluide inclut une première unité d'amenée de fluide qui amène le fluide dans le premier groupe de canaux d'endoscope, et une deuxième unité d'amenée de fluide qui amène le fluide dans le deuxième groupe de canaux d'endoscope ;
l'unité de mesure inclut une première unité de mesure (47) qui mesure les débits du fluide s'écoulant à travers les premiers canaux d'endoscope, et une deuxième unité de mesure (183) qui mesure la pression du fluide s'écoulant à travers le deuxième groupe de canaux d'endoscope ; et
la première unité d'amenée de fluide et la première unité de mesure forment un premier système de mesure (181) ; et
la deuxième unité d'amenée de fluide et la deuxième unité de mesure forment un deuxième système de mesure (182).
